# EUROPÄISCHE PATENTSCHRIFT

(11) **EP 1 141 291 B1**
(45) Veröffentlichungstag und Bekanntmachung des Hinweises auf die Patenterteilung: **25.03.2009**
(21) Anmeldenummer: 99967990.5
(22) Anmeldetag: 29.12.1999
(51) Int. Cl.: C12N 15/12, C12N 15/55, C12N 15/10, C12N 15/62, C12N 15/81, C12N 9/16, C07K 14/47, C07K 14/72, C07K 14/82, G01N 33/566, G01N 33/68

(54) **METHODE ZUR ZELLULÄREN HIGH-THROUGHPUT-DETEKTION VON NUKLEÄREN REZEPTOR-LIGANDEN-INTERAKTIONEN**
METHOD FOR THE CELLULAR HIGH-THROUGHPUT-DETECTION OF NUCLEAR RECEPTOR LIGAND INTERACTIONS
METHODE DE DETECTION CELLULAIRE A HAUT RENDEMENT D'INTERACTIONS ENTRE DES RECEPTEURS NUCLEAIRES ET DES LIGANDS

(30) Priorität: 30.12.1998 DE 19860834
(43) Veröffentlichungstag der Anmeldung: 10.10.2001
(73) Patentinhaber: Forster, Hansjörg, Dr., 37269 Eschwege (DE); Zimmermann, André, 79102 Freiburg (DE)
(72) Erfinder: SIPPEL, Albrecht E., 79104 Freiburg (DE); ZIMMERMANN, André, 79102Freiburg (DE)
(74) Vertreter: Weiss, Wolfgang
(86) Internationale Anmeldenummer: PCT/EP1999/010461
(87) Internationale Veröffentlichungsnummer: WO 2000/040717

(56) Entgegenhaltungen:
- WO-A-94/28914
- US-A- 5 776 689
- BRODER Y C ET AL: "THE RAS RECRUITMENT SYSTEM, A NOVEL APPROACH TO THE STUDY OF PROTEIN-PROTEIN INTERACTIONS" CURRENT BIOLOGY,GB,CURRENT SCIENCE,, Bd. 8, Nr. 20, Oktober 1998 (1998-10), Seiten 1121-1124, XP000867036 ISSN: 0960-9822
- A ARONHEIM: "Improved eficciency Sos recruitment system: expression of the mammalian GAP reduces isolation of Ras GTPase false positives" NUCLEIC ACIDS RESEARCH, Bd. 25, Nr. 16, 15. August 1997 (1997-08-15), Seiten 3373-3374, XP002136255 IRL PRESS LIMITED,OXFORD,ENGLAND
- ARONHEIM A ET AL: "ISOLATION OF AN AP-1 REPRESSOR BY A NOVEL METHOD FOR DETECTING PROTEIN-PROTEIN INTERACTIONS" MOLECULAR AND CELLULAR BIOLOGY,US,AMERICAN SOCIETY FOR MICROBIOLOGY, WASHINGTON, Bd. 17, Nr. 6, Juni 1997 (1997-06), Seiten 3094-3102, XP000867026 ISSN: 0270-7306
- ARONHEIM A ET AL: "MEMBRANE TARGETING OF THE NUCLEOTIDE EXCHANGE FACTOR SOS IS SUFFICIENT FOR ACTIVATING THE RAS SIGNALING PATHWAY" CELL,US,CELL PRESS, CAMBRIDGE, NA, Bd. 78, 23. September 1994 (1994-09-23), Seiten 949-961, XP002037807 ISSN: 0092-8674
- ARONHEIM A ET AL: "CHP, A HOMOLOGUE OF THE GTPASE CDC42HS, ACTIVATES THE JNK PATHWAY AND IS IMPLICATED IN REORGANIZING THE ACTIN CYTOSKELETON" CURRENT BIOLOGY,GB,CURRENT SCIENCE,, Bd. 8, Nr. 20, Oktober 1998 (1998-10), Seiten 1125-1128-1128, XP000867010 ISSN: 0960-9822
- J. SCHLESSINGER: "How receptor tyrosine kinases activate RAS" TRENDS IN BIOCHEMICAL SCIENCES, Bd. 18, Nr. 08, August 1993 (1993-08), Seiten 273-275, XP002136256 ELSEVIER, AMSTERDAM, NL in der Anmeldung erwähnt

## Beschreibung

Die Erfindung bezieht sich auf das Gebiet der Molekularbiologie. Sie betrifft insbesondere Assayverfahren, die dazu dienen, spezifische Interaktionen zwischen einem Ligand und einem nukleären Rezeptor zu detektieren, und zielt u.a. darauf ab, neuartige, funktionelle Liganden für nukleäre Rezeptoren zu finden wie auch eine Ligandenbindungsfunktion, die für nukleäre Rezeptoren charakteristisch ist, bei Polypeptiden oder Proteinen, bei denen eine solche Funktion vermutet wird, gegebenenfalls nachzuweisen. In diesem Zusammenhang betrifft die Erfindung auch Fusionsproteine, Nukleinsäuren, die diese Fusionsproteine kodieren, Vektoren, die diese Nukleinsäuren enthalten, Zellen, die diese Fusionsproteine enthalten, Kits, die sämtlich für die erfindungsgemäßen Assayverfahren oder in Zusammenhang mit diesen eingesetzt werden können, sowie Liganden für einen Bindungsabschnitt eines Rezeptors, Verbindungen, die in der Lage sind, eine Bindungsaktivität eines Ligandenbindungsabschnitts eines Rezeptors gegenüber einem Liganden zu verändern, und Polypeptide oder Proteine, die eine Ligandenbindungsfunktion eines Rezeptors aufweisen, die sämtlich mittels der erfindungsgemäßen Verfahren, und insbesondere der erfindungsgemäßen Assayverfahren erhalten bzw. identifiziert werden können.

Die Familie der nukleären Rezeptoren unterscheidet sich von anderen Rezeptorfamilien (z.B. der 7-Transmembranrezeptorfamilie oder der Tyrosinkinaserezeptorfamilie) durch die Tatsache, daß sie weder eine Transmembrandomäne noch ein sonstwie geartetes Membranlokalisierungs- oder -verankerungssignal besitzen. Ohne einen gebundenen Liganden liegen sie in inaktiver Form entweder im Zytoplasma und/oder im Zellkern vor. Gemeinsam ist allen Mitgliedern dieser Rezeptorfamilie, daß sie bei der Bindung ihres Liganden eine Konformationsänderung durchlaufen und dadurch in eine sogenannte "aktive" bzw. anders effektive Form übergehen. Zur Familie der nukleären Rezeptoren gehören u.a. die Steroid-Rezeptoren (Evans, Science, 240:889-95, 1988), die Orphan-Rezeptoren (Bargmann, Cell, 90: 585-587, 1997), der Vitamin D-Rezeptor, der Thyroxin-, Rezeptor, der Dioxin-Rezeptor, die Retinsäure-Rezeptoren und viele andere Rezeptoren (Kastner et al., Cell, 83: 859-869, 1995). Nukleäre Rezeptoren spielen eine wichtige Rolle bei einer Vielzahl ganz unterschiedlicher biologischer Prozesse, wie z.B. bei der Entwicklung von Organismen, der Zelldifferenzierung, der Zellteilung, der Genregulation und vor allem auch bei der Entstehung von Krebs (Seed, Nature Medicine, 4: 1004-1005, 1998).

Viele medizinische Therapien nutzen die Möglichkeit, mit Hilfe bestimmter Pharmazeutika in Prozesse einzugreifen, die von nukleären Rezeptoren gesteuert werden. Die dabei verwendeten Pharmazeutika wirken als Agonisten oder Antagonisten eines für einen bestimmten nukleären Rezeptor spezifischen, in der Natur vorkommenden Liganden.

Aus diesen und anderen Gründen besteht großes Interesse daran, Rezeptor-Liganden-Interaktionen auf molekularer Ebene detektieren und studieren zu können.

Es existieren bereits mehrere Verfahren zur Detektion von nukleärer Rezeptor-Liganden-Interaktionen. Die meisten dieser Verfahren nutzen zur Detektion einer solchen Interaktion die Tatsache, daß der mit dem Liganden komplexierte Rezeptor in der Lage ist, spezifisch an DNA zu binden und in Zellen ein sogenanntes Reportergen zu aktivieren oder zu inaktivieren (US-Patente 4,981,784 und 5,643,720). Der Nachteil solcher Verfahren beruht u.a. auf dem z.T. aufwendigen Nachweis der Reportergenaktivität oder dem Aufwand beim Umgang mit den verwendeten Vertebratenzellen.

In einem anderen Ansatz wird die Rezeptor-Liganden-Interaktion *ex vivo* detektiert, also außerhalb eines lebenden Systems, wobei entweder der Rezeptor oder der Ligand auf eine Matrix aufgebracht und mit einer Lösung, die den Liganden oder Rezeptor enthält, umspült wird. Auch hier ist der Aufwand zur Gewinnung und Aufbringung jedes einzelnen Rezeptors oder Liganden auf die entsprechende Matrixoberfläche überaus hoch. Zudem ergibt sich hierbei ein Problem bei der Übertragung der gewonnenen Resultate auf die Verhältnisse in der Zelle, da die zellulären Bedingungen von den ex vivo-Bedingungen erheblich abweichen können. Ein weiteres gravierendes Problem besteht vor allem in der fehlenden Zugriffsmöglichkeit auf die genetische Information der in Screens oder High-Throughput-Assays detektierten, neuen Rezeptorvarianten.

Die der Erfindung zugrundeliegende Aufgabe besteht darin, alternative Assays, die dazu geeignet sind, spezifische Interaktionen zwischen einem Ligand und einem nukleären Rezeptor *in vivo* zu detektieren, bereitzustellen, die u.a. die Vorteile bieten, Liganden-Rezeptor-Wechselwirkungen schneller als im Stand der Technik möglich detektieren zu können und auch mittels einfacher handzuhabender Zellen, wie prokaryotischen Zellen oder Hefezellen, ausgeführt werden zu können. Darüber hinaus besteht aufgrund der Assaygestaltung stets eine unmittelbare Zugriffsmöglichkeit auf die einer Rezeptorväriante zugrundeliegende genetische Information.

Weitere Aufgaben der Erfindung bestehen darin, Fusionsproteine, Nukleinsäuren, Vektoren, Zellen und Kits, die sämtlich für die erfindungsgemäßen Assayverfahren oder in Zusammenhang mit diesen eingesetzt werden können, sowie Liganden für einen Bindungsabschnitt eines Rezeptors, Verbindungen, die in der Lage sind, eine Bindungsaktivität eines Ligandenbindungsabschnitts eines Rezeptors gegenüber einem Liganden zu verändern, und Polypeptide oder Proteine, die eine Ligandenbindungsfunktion eines Rezeptors aufweisen, die sämtlich mittels der erfindungsgemäßen Verfahren, und insbesondere der erfindungsgemäßen Assayverfahren erhalten bzw. identifiziert werden können, bereitzustellen.

Die der Erfindung zugrundeliegenden Aufgaben werden durch die in den Ansprüchen u.a. definierten Verfahren, insbesondere Assay-Verfahren, Fusionsproteine, Nukleinsäuren, Vektoren, Zellen, Kits, Liganden, Verbindungen, Polypeptide und Proteine erfüllt.

Die vorliegende Erfindung beruht auf den folgenden Erkenntnissen:
1. Die Aktivität eines Proteins, das in der Lage ist, einen sich an ein Ras-Protein anschließenden Signalweg in einer Zelle zu aktivieren, kann durch die Fusion an einen nukleären Rezeptor und/oder Teile von solchen in Abhängigkeit von der Ligandenbindung an den Rezeptor oder Rezeptoranteil gesteuert werden.
2. Zur Aktivierung verschiedener ras-Signaltransduktionswege ist die Membranlokalisierung bestimmter Komponenten der Signalwege notwendig (Schlessinger, TIBS, 18: 273-275, 1993). Befinden sich diese Komponenten in Fusion mit einem nukleären Rezeptor und/oder mit Teilen von solchen, wie unter 1. erläutert, so kann bei zusätzlicher Anfügung einer weiteren Domäne, die eine Membranlokalisierung des Fusionsprodukts vermittelt, in einer Zelle ein System geschaffen werden, bei dem die Aktivität der Komponente zur Aktivierung des ras-Signaltransduktionsweges gezielt am Wirkort, d.h. an der Membran, nur in Gegenwart oder, alternativ, nur in Abwesenheit eines Liganden für den nukleären Rezeptor wirksam werden kann.
3. Im Stand der Technik sind Zellen bekannt, in denen ein ras-Signaltransduktionsweg auf Ebene des dafür spezifischen Ras-Proteins oder eines für das Ras-Protein spezifischen Guaninnukleotid-Austauschfaktors zumindest unter bestimmten Bedingungen inaktiviert werden kann. Führt man in eine solche Zelle das vorstehend unter 2. erläuterte Fusionsprotein, das eine ras-Aktivität aufweist, die den genannten ras-Signaltransduktionsweg aktivieren kann, ein, so erhält man eine Zelle, bei der der zumindest unter bestimmten Bedingungen inaktive zelleigene ras-Signaltransduktionsweg durch die Aktivität der entsprechend aktiven Komponente des Fusionsproteins aktiviert werden kann - allerdings nur in Gegenwart oder, alternativ, in Abwesenheit eines Liganden für den nukleären Rezeptorabschnitt.

Man erhält auf diese Weise eine Zelle, in der der oder ein bestimmter ras-Signaltransduktionsweg nur in Abhängigkeit von einer Ligandenbindung, d.h. bei erfolgter Ligandenbindung oder, alternativ, bei fehlender Ligandenbindung, an den nukleären Rezeptorabschnitt des vorstehend erläuterten Fusionsproteins aktiviert werden kann. Diese Zelle ermöglicht die Etablierung eines in vivo-Assayver-fahrens, welches anhand des Nachweises einer gegebenenfalls erfolgten Aktivierung des speziellen ras-Signaltransduktionswe-ges, ggf. indirekt über an oder in der Zelle nachweisbare spezifische Auswirkungen, wie Zellwachstum, die Detektion von Interaktionen zwischen einem nukleären Rezeptor und einem dafür spezifischen Liganden ermöglicht.

Zum klareren Verständnis der Lehre dieser Unterlagen sei angefügt, daß im vorliegenden Zusammenhang unter dem Begriff "Ligand" nur solche Bindungspartner für Rezeptoren und insbesondere nukleäre Rezeptoren verstanden werden sollen, die bei einer Bindung an den Ligandenbindungs- oder Rezeptorabschnitt eines solchen Rezeptors eine Konformationsänderung hervorrufen, die die dritte Domäne in die Lage versetzt oder, alternativ, daran hindert, ihre Aktivität zur Aktivierung eines sich an ein Ras-Protein anschließenden Signalwegs in einer Zelle auszuüben. Bei Abdissoziierung des Liganden von dem Ligandenbindungs- oder Rezeptorabschnitt, d.h. bei fehlender Ligandenbindung, ist die dritte Domäne bei der vorstehend erstgenannten Variante dementsprechend nicht in der Lage, ihre Aktivität zur Aktivierung eines sich an ein Ras-Protein anschließenden Signalwegs in einer Zelle auszuüben. Bei der zweiten Variante liegt allein in diesem Falle bei der dritten Domäne die Fähigkeit vor, den sich an ein Ras-Protein anschließenden Signalweg in einer Zelle zu aktivieren. Dies kann insbesondere Konformationsänderungen umfassen, wie sie *in vivo* bei der Bindung eines natürlichen Liganden erfolgen. Im Falle nukleärer Rezeptoren, wie sie bereits im Vorangegangenen erläutert wurden, bewirkt die Konformationsänderung bei Ligandenbindung, wie erwähnt, eine Aktivierung und zwar wahrscheinlich, wie aufgrund von tragfähigen Hinweisen vermutet wird, durch eine durch die Konformationsänderung bewirkte Abdissoziierung eines Multiproteinkomplexes, der bei fehlender Ligandenbindung in fester Assoziierung mit dem Rezeptor vorliegt.

Es werden jedoch auch Fälle in Betracht gezogen, bei denen die Konformationsänderungen nicht oder nur teilweise den bei Bindung eines *in vivo* in der Zelle vorkommenden Liganden erfolgenden Konformationsänderungen entsprechen.

Die Bedeutung des Begriffes "nukleärer Rezeptor" soll sich im vorliegenden Zusammenhang zudem zusätzlich auch auf z.B. virale (einschließlich retrovirale), nicht-membranständige Rezeptoren erstrecken, die ebenfalls die Eigenschaften aufweisen, ohne gebundenen Liganden in inaktiver Form vorzuliegen, jedoch bei Bindung ihres Liganden eine Konformationsänderung zu durchlaufen und dadurch in eine sogenannte "aktive" Form überzugehen. In Zusammenhang mit dieser Variante der Erfindung ist zudem essentiell, daß bei Integration der Ligandenbindungsdomäne eines solchen viralen Rezeptors in ein erfindungsgemäßes Fusionsprotein und bei fehlender Bindung von Ligand die dritte Domäne ihre Aktivität zur Aktivierung eines sich an ein Ras-Protein anschließenden Signalwegs in einer Zelle trotz Membranlokalisierung nicht ausüben kann; d.h., die Aktivität der dritten Domäne zur Aktivierung eines sich an ein Ras-Protein anschließenden Signalwegs erfordert bei dieser Variante zwingend die Bindung eines Liganden an den von dem viralen Rezeptor stammenden Ligandenbindungsabschnitt.

Alternativ ist auch im Falle viraler Rezeptoren die Möglichkeit vorgesehen, daß solche Rezeptoren ohne gebundenen Liganden in inaktiver Form vorliegen, jedoch bei Bindung ihres Liganden eine Konformationsänderung durchlaufen und dadurch in eine sogenannte "aktive" Form übergehen. In Zusammenhang der Erfindung kann bei dieser Variante die dritte Domäne bei Integration der Ligandenbindungsdomäne eines entsprechenden viralen Rezeptors in ein erfindungsgemäßes Fusionsprotein und bei Bindung von Ligand ihre Aktivität zur Aktivierung eines sich an ein Ras-Protein anschließenden Signalwegs in einer Zelle trotz Membranlokalisierung nicht ausüben; diese Aktivität erfordert die Abdissoziierung des Liganden, also eine Form des Fusionsproteins ohne gebundenen Liganden.

Im Rahmen der Erfindung und insbesondere im Falle viraler Rezeptoren sind neben dem vorstehend erläuterten Mechanismus zur "Aktivierung" der dritten Domäne in Form einer Konformationsänderung, die zur Abdissoziierung eines Multiproteinkomplexes führt, somit auch andere Aktivierungsmechanismen denkbar, die jedoch stets eine durch die Ligandenbindung oder, alternativ, Abdissozüerung von Ligand bewirkte Konformationsänderung innerhalb des Fusionsproteins voraussetzen werden.

Von den hier synonym verwendeten Begriffen "ras-Signalweg" oder "sich an ein Ras-Protein anschließender Signalweg" werden auch die sogenannten ras-ähnlichen Signalwege mitumfaßt, die von diversen weiteren Mitgliedern der Ras-Familie gesteuert werden. Unter den Mitgliedern der Ras-Familie gibt es solche, die trotz Ursprungs aus unterschiedlichen Organismen ein und denselben Signaltransduktionsweg in einer gewählten Zielzelle aktivieren können. Ein Beispiel hierfür ist das humane Ha-Ras (L61), das in der Lage ist, auch einen ras-Signalweg in *Saccharomyces cerevisiae* zu aktivieren, der auf den Zellzyklus einwirkt und dessen Aktivierung für eine Vermehrung der Hefezellen essenziell ist. Andere Mitglieder der Ras-Familie sind nur in der Lage, einen einzigen, für sie spezifischen Signalweg zu aktivieren.

Eine Reihe von Mitgliedern der Ras-Familie, wie das vorstehend erwähnte Ha-Ras (L61), aktiviert Signalwege, die auf den Zellzyklus einwirken und deren Aktivierung über die Aktivierung spezieller Transkriptionsfaktoren für die Zellvermehrung essenziell sind. Andere derartige Ras-Proteine aktivieren Signalwege, die spezifisch zur Aktivierung jeweils eines einer Vielzahl von Transkriptionsfaktoren, die für andere Gene als jene des Zellzyklus spezifisch sind, führen. Im vorliegenden Kontext ist allen ras-Signalwegen gemeinsam, daß sie für ihre Aktivierung ein an der Zellmembran vorliegendes aktives Ras-Protein erfordern, wobei das Ras-Protein gegebenenfalls für seine Aktivität die gleichzeitige Anwesenheit eines Guaninnukleotid-Austauschfaktors an der Zellmembran benötigt.

Wird in diesen Unterlagen auf eine Inaktivierung eines ras-Signalwegs oder eines ras-ähnlichen Signalwegs Bezug genommen, so wird darunter stets eine Inaktivierung auf Ebene des Ras-Proteins und/oder eines dafür spezifischen Guaninnukleotid-Austauschfaktors verstanden. Die genannte Signalwegsinaktivierung tritt in einer Zelle im vorliegenden Zusammenhang bevorzugt nur unter bestimmten Umweltbedingungen, wie Temperatur, auf, kann also durch gezielte Einstellung von Umweltbedingungen induziert und wieder aufgehoben werden.

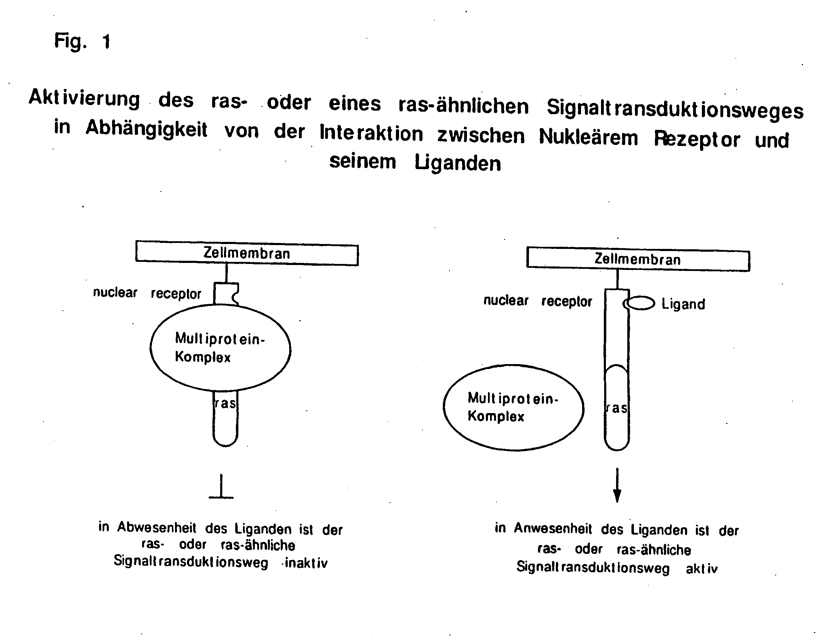

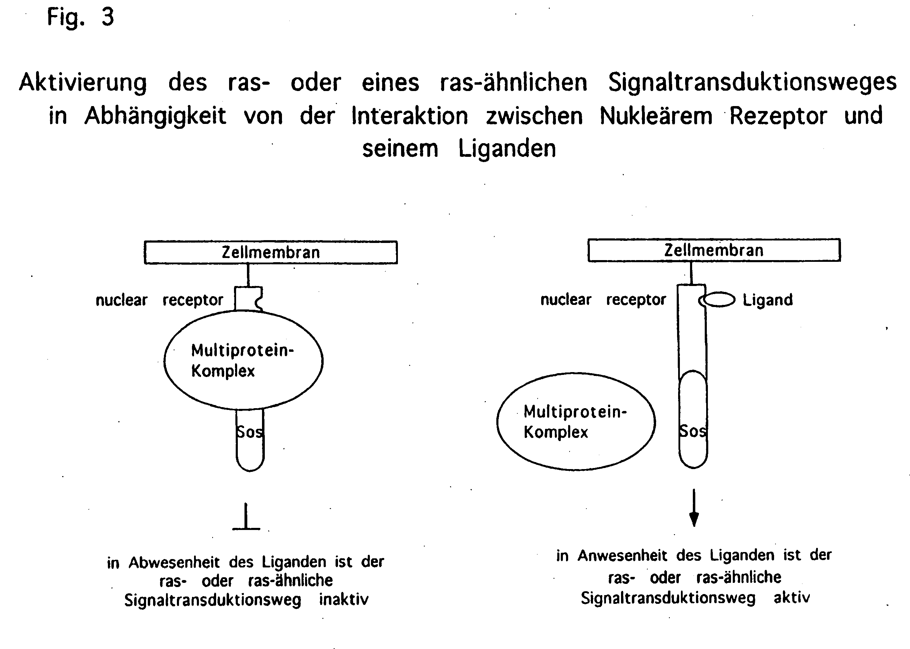

Wesentliche Voraussetzung der erfindungsgemäßen Assaysysteme ist, wie erläutert, die Expression eines Fusionsproteins mit bestimmten Eigenschaften in einem geeigneten Zellsystem. Wie auch in den Figuren 1 und 3 schematisch darstellt, umfaßt dieses Fusionsprotein, das ebenfalls einen Teil der Erfindung darstellt, Domänen bzw. Abschnitte, die dem Fusionsprotein folgende drei Funktionen verleihen oder im Falle der im Folgenden genannten zweiten Funktion verleihen sollen:
1. Membranlokalisierung, z.B. durch ein Membranlokalisierungssignal, eine Transmembrandomäne und/oder irgend einen anderen Proteinanteil, der Membranlokalisierung vermittelt,
2. Ligandenbindungsfunktion eines nukleären Rezeptors, z.B. durch das Vorsehen der Sequenz eines vollständigen nukleären Rezeptors und/oder von Teilen eines solchen,
3. Fähigkeit zur Aktivierung eines ras- oder ras-ähnlichen Signaltransduktionsweges.
Weiteres wesentliches Merkmal ist, daß bei fehlender Bindung oder, alternativ, bei Bindung von Ligand an die zweite Domäne mit Ligandenbindungsfunktion die dritte Domäne ihre Aktivität zur Aktivierung eines sich an ein Ras-Protein anschließenden Signalweges in einer Zelle trotz Lokalisierung an der Zellmembran nicht ausüben kann. Diese Aktivität erfordert in der vorstehend erstgenannten Variante das Vorliegen eines an die zweite Domäne gebundenen Liganden, in der letztgenannten Variante das Abdissoziieren von an die zweite Domäne gebundenem Ligand.

Aufgrund der vorstehend kurz erwähnten und nachfolgend näher erläuterten Hinweise aus diversen Experimenten an nukleären Rezeptoren wird angenommen, daß in einer bevorzugten Ausführungsform bei fehlender Bindung von Ligand an die zweite Domäne die dritte Domäne mit der Aktivierungsfunktion durch einen sich an das Fusionsprotein anlagernden Multiproteinkomplex so komplexiert werden kann, daß die letztere nicht in der Lage ist, ihre Aktivität zur Aktivierung eines ras- oder ras-ähnlichen Signaltransduktionsweges in einer Zelle auszuüben. Ein analoger Mechanismus ist als eine Möglichkeit auch bei der anderen Variante denkbar, d.h. Komplexierung und damit Inaktivierung der dritten Domäne allerdings bei Ligandenbindung, wobei in diesem Falle die Abdissoziierung des Liganden zu einer Abdissoziierung des Multiproteinkomplexes führt und damit die dritte Domäne in die Lage versetzt, ihre Aktivität zur Aktivierung eines sich an ein Ras-Protein anschließenden Signalweges auszuüben.

Infolge diverser Experimente an nukleären Rezeptoren wird angenommen, daß diese in der Zelle ohne gebundenen Ligand als inaktiver Multiproteinkomplex vorliegen (Pratt, Endocr. Rev., 18: 306-60, 1997), welcher aus sogenannten "heat shock"-Proteinen (HSPs) bestehen kann, die in der Zelle exprimiert werden. Es wird angenommen, daß die Anlagerung des Multiproteinkomplexes bei einem natürlich vorkommenden nukleären Rezeptor in der Nähe, ggf. unmittelbaren Nähe der Ligandenbindungsstelle oder ggf. auch in einem damit überlappenden Bereich erfolgt. Wenn mittels der erfindungsgemäßen Assays jedoch die Ligandenbindungsfunktion eines mutierten oder künstlich erzeugten Ligandenbindungsabschnitts überprüft werden soll, kann es gegebenenfalls erforderlich sein, zusätzlich einen oder mehrere Abschnitte nukleärer Rezeptoren vorzusehen, von denen bekannt ist oder bezüglich jener nachweisbar ist, daß sie die Multiproteinkomplexbindung vermitteln, die jedoch keine Ligandenbindungsfunktion mehr aufweisen. In diesem Falle kann die zweite Domäne als chimäre Sequenz von Aminosäuresequenzen verschiedenen Ursprungs vorgesehen werden. Alternativ kann dieser zusätzliche Sequenzabschnitt aber auch als weitere, vierte Domäne in geeigneter Anordnung innerhalb des Fusionsproteins vorgesehen werden.

Das zusätzliche Vorsehen eines solchen zusätzlichen Sequenzabschnitts kann auch im Falle von aus viralen Rezeptoren stammenden Ligandenbindungsabschnitten in Betracht gezogen werden, sofern eine Ligandenbindung an den Ligandenbindungsabschnitt eine solche Konformationsänderung bewirken kann, daß ein zuvor über den zusätzlichen Sequenzabschnitt (mit) gebundener Multiproteinkomplex abdissoziiert.

Im Rahmen der Erfindung sollen jedoch alternative Mechanismen zur Aktivierung der dritte Domäne infolge einer Ligandenbindung oder, alternativ, aufgrund fehlender Ligandenbindung aufgrund einer dadurch verursachten Konformationsänderung ausdrücklich mitumfaßt sein.

Die vorstehend erstgenannte Funktion bewirkt, daß das Fusionsprotein an die Membran gelangt und damit an den Wirkort desjenigen Teils des Fusionsproteins, welcher für die dritte Funktion verantwortlich ist. Die Ausübung der dritten Funktion steht in direkter Abhängigkeit von der als zweites genannten Ligandenbindungsfunktion des Fusionsproteins, und zwar von der Anwesenheit eines Liganden, der mit dieser Domäne interagiert.

Wie erwähnt, wird angenommen, daß nukleäre Rezeptoren in der Zelle in Abwesenheit von Ligand als inaktive Multiproteinkomplexe vorliegen. Gleiches soll in einer bevorzugten Ausführungsform für das erfindungsgemäße Fusionsprotein, das in seiner zweiten Domäne die Sequenz eines solchen nukleären Rezeptors oder von Abschnitten eines solchen enthält, gelten. Der Multiproteinkomplex kann dabei insbesondere zelleigene "heat shock"-Proteine (hsp) umfassen. Auch in einem erfindungsgemäßen Fusionsprotein mit einer zweiten Domäne, die einen mutierten oder künstlichen, z.B. mittels "molecular modelling" gestalteten Ligandenbindungsabschnitt mit einer lediglich vermuteten Ligandenbindungsfunktion enthält, ist bei dieser Ausführungsform die Multiproteinkomplexbindung im zellulären Kontext in Abwesenheit von Ligand ein wesentliches Merkmal. Gegebenenfalls muß hierfür innerhalb des Fusionsproteins, wie erläutert, ein zusätzlicher Proteinabschnitt, der die Multiproteinkomplexbindung in Abwesenheit von Ligand vermittelt, z.B. aus einem nukleären Rezeptor, vorgesehen werden. In beiden Fällen verhindert dieser Multiproteinkomplex die Aktivität der dritten Domäne oder, synonym, ras- oder ras-ähnlichen Signaltransduktionskomponente. Bindet jedoch ein spezifischer Ligand an den zugehörigen nukleären Rezeptorabschnitt, dissoziiert wie bei den *in vivo* gefundenen nukleären Rezeptoren der Multiproteinkomplex von der Rezeptordomäne ab und die ras- oder ras-ähnliche Signaltransduktionskomponente wird aktiv (siehe Fig. 1 und Fig. 3; "nuclear receptor" = nukleärer Rezeptor).

Im zellulären Kontext wird nun durch Einwirkung der aktiven Signaltransduktionskomponente ein ras- oder ras-ähnlicher Signaltransduktionsweg aktiviert. Verwendet man eine Zelle, bei der dieser ras- oder ras-ähnliche Signalweg in Abwesenheit des Fusionsproteins aufgrund von Mutationen zumindest unter bestimmten Bedingungen nicht aktiviert ist, kann eine solche allein durch das Fusionsprotein vermittelte Aktivierung über phänotypische Veränderungen, z.B. Wachstum oder Gen- bzw. Reportergenaktivität, in der Zelle detektiert werden.

In bevorzugten Ausführungsformen dieser Erfindung umfaßt die Membranlokalisierungsdomäne die Aminosäuresequenz eines Farnesylierungssignals, Myristylierungssignals oder Prenylierungssignals oder ist davon, z.B. durch Aminosäureaustausch, -modifizierung, -insertion oder -deletion, abgeleitet.

Im Bereich der Membranlokalisierungsdomäne oder als ein zusätzlicher, insbesondere N-terminal angeordneter Sequenzabschnitt kann ferner eine Signalsequenz vorgesehen werden, die zwar nicht der Membranverankerung des Fusionsproteins an sich dient, jedoch bewirkt, daß das Fusionsprotein nach seiner Expression mit höherer Effizienz an die Zellmembran transportiert wird. Die daraus resultierende höhere Konzentration von Fusionsprotein in unmittelbarer Nähe der Membran führt in der Folge zu einer höheren Einbaurate des Fusionsproteins in die Zellmembran aufgrund der Membranlokalisierungsdomäne. Solche Signalsequenzen werden bevorzugt speziell auf den Zelltyp, in dem das Fusionsprotein exprimiert werden soll, abgestimmt verwendet, da beispielsweise in Hefe wirksame Signalsequenzen in Säugetierzellen nur mit geringerer Effizienz wirksam sind und umgekehrt. Beispiele für solche Signalsequenzen sind Signalsequenzen von hefeeigenen GPCRs (G-Protein gekoppelten Rezeptoren) oder von hefeeigener Invertase (SUC2) zur bevorzugten Verwendung in Fusionsproteinen, die in Hefezellen exprimiert werden sollen.

Die Aminosäuresequenz der zweiten Domäne mit Ligandenbindungsfunktion eines nukleären Rezeptors kann die Aminosäuresequenz eines in der Natur vorkommenden nukleären Rezeptors, wie eines Steroid-Rezeptors, Orphan-Rezeptors, Vitamin-Rezeptors, beispielsweise Vitamin D-Rezeptors, Thyroxin-Rezeptors oder Retinsäurerezeptors, umfassen oder davon, z.B. durch Aminosäureanfügung, -austausch, -modifizierung, -insertion oder -deletion, abgeleitet sein. Alternativ kann die zweite Domäne einen in der Natur nicht vorkommenden, beispielsweise durch "molecular modelling" erzeugten synthetischen Rezeptorabschnitt mit gegebenenfalls zunächst nur vermuteter Ligandenbindungsfunktion umfassen.

Die dritte Domäne vermag bevorzugt, ras-Signaltransduktionswege zu aktivieren, die auf den Zellzyklus einwirken und deren Aktivierung für die Zellvermehrung essenziell ist. Alternativ und ebenso bevorzugt wirkt sie auf einen der Ras-Signalwege ein, die der Aktivierung von Transkriptionsfaktoren für Gene dienen, die für die Zellvermehrung nicht essenziell sein müssen.

Die dritte Domäne kann die Aktivität eines aktiven und insbesondere eines konstitutiv aktiven Ras-Proteins aufweisen. Konstitutiv aktive Ras-Proteine zeigen Aktivität unabhängig von der Anwesenheit von Guaninnukleotid-Austauschfaktor-Molekülen, die diverse andere Ras-Proteine für ihre Aktivität benötigen. Zu diesem Zweck kann die dritte Domäne beispielsweise die Aminosäuresequenz eines aktiven oder konstitutiv aktiven Ras-Proteins, das in der Natur vorkommt, z.B. dem humanen Ha-Ras (L61), oder von Teilen davon umfassen. Oder sie kann Aminosäuresequenzen umfassen, die von derartigen Sequenzen, z.B. durch Aminosäureanfügung, -austausch, -modifizierung, -insertion oder -deletion, abgeleitet sind.

Alternativ kann die dritte Domäne die Aktivität eines funktionellen Guaninnukleotid-Austauschfaktors aufweisen. In dieser Hinsicht kann die Aminosäuresequenz der dritten Domäne ebenfalls beispielsweise Sequenzen von in der Natur vorkommenden Guaninnukleotid-Austauschfaktoren oder Teilsequenzen davon umfassen oder sie kann davon, z.B. durch Aminosäureanfügung, -austausch, -modifizierung, -insertion oder -deletion, abgeleitet sein. In einer bevorzugten Ausführungsform ist die Aminosäuresequenz der dritten Domäne abgeleitet von der Aminosäuresequenz des CDC25-Proteins aus *Saccharomyces cerevisiae,* eines SOS-Proteins aus einem Säugetier oder eines aus einem beliebigen Organismus abgeleiteten SOS-ähnlichen Proteins.

In einer bevorzugten Ausführungsform dieser Erfindung, die in den Figuren 1 und 3 schematisch erläutert ist, sind die einzelnen Domänen innerhalb des Fusionsproteins in Richtung vom N-Terminus zum C-Terminus in der Abfolge erste Domäne (Membranlokalisierungsdomäne), zweite Domäne (Domäne mit Ligandenbindungsfunktion), dritte Domäne (ras- oder ras-ähnliche Signaltransduktionskomponente) angeordnet. Die Anordnung der einzelnen Domänen kann aber auch anders sein. Beispielhaft sei hier eine Abfolge vom N-Terminus in Richtung des C-Terminus in der Reihenfolge dritte Domäne, zweite Domäne, erste Domäne aufgeführt.

Gegebenenfalls kann das erfindungsgemäße Fusionsprotein auch weitere Domänen oder Proteinabschnitte mit oder ohne Funktion umfassen, solange die vorstehend erläuterten Funktionen davon unbeeinträchtigt oder im wesentlichen unbeeinträchtigt bleiben.

Darüber hinaus umfaßt die Erfindung DNA-Moleküle, die die erfindungsgemäßen Fusionsproteine kodieren, sowie Vektoren, insbesondere Plasmide, Cosmide, Viren- oder Phagengenome, die mindestens eines dieser DNA-Moleküle umfassen. Besondere erfindungsgemäße Vektoren sind zur Transformation oder Transfektion von Wirtszellen oder zur Expression mindestens eines erfindungsgemäßen Fusionsproteins geeignet. Zu dem letztgenannten Zweck steht ein erfindungsgemäßes DNA-Molekül in dem Vektor unter Kontrolle eines in einer Wirtszelle funktionsfähigen Promotors, der die Expression ermöglicht und steuert.

Die Herstellung der erfindungsgemäßen Fusionsproteine, DNA-Moleküle und Vektoren kann nach im Stand der Technik bekannten Protokollen erfolgen (siehe z.B. Sambrook, J., Fritsch, E.F., Maniatis, T. (1989), Molecular Cloning. A Laboratory Handbook, Cold Spring Harbor Laboratory, New York; Current Protocols in Molecular Biology (1991)). Auch wenn die Fusionsproteine grundsätzlich vollsynthetisch aus einzelnen Aminosäure-Derivaten hergestellt werden können, wozu im Stand der Technik diverse Verfahren zur Verfügung stehen, werden sie üblicherweise über die Expression der entsprechenden Gene in Zellen produziert. Dabei kann das Gen für das Fusionsprotein extrachromosomal oder in das Genom der Wirtszelle integriert vorliegen. Die Klonierung der Gene für die Fusionsproteine ausgehend von bekannten Genabschnitten, die Proteinabschnitte mit den erforderlichen oder im Falle des Ligandenbindungs- oder Rezeptorabschnitts auch vorerst nur vermuteten Funktionen kodieren, gehört ebenso zu den Standardfähigkeiten eines Fachmanns, wie die Konstruktion von Vektoren, wie Transkriptions- oder Transfektionsvektoren oder auch Expressionsvektoren, in denen das Gen in funktionaler Verknüpfung mit einem in der Produktionszelle wirksamen Promotor vorliegt, die Transformation oder Transfektion von Wirtszellen wie auch die Züchtung der transformierten bzw. transfizierten Wirtszellen zur Produktion des Proteins. Die Isolierung und Reinigung der erfindungsgemäßen Fusionsproteine kann durch Einsatz herkömmlicher Verfahren, wie Fällung, Einsatz diverser Chromatographieverfahren, wie Gelfiltration, Affinitätschromatographie u.s.w. erfolgen. Insbesondere erlaubt die Affinitätschromatographie beispielsweise bei Verwendung von an die Matrix gebundenen spezifischen Antikörpern, die gegen eine Determinante eines bezüglich der Wirtszelle heterologen Abschnitts des Fusionsproteins gerichtet sind, eine selektive Bindung nur des Fusionsproteins. Alternativ kann das Fusionsprotein beispielsweise auch als Vorläuferprotein exprimiert werden, das eine zusätzliche Domäne mit einer spezifischen Bindungseigenschaft an eine bestimmte Affinitätssäule aufweist. Nach Bindung und darauffolgender Elution von der Affinitätssäule kann die zusätzliche Domäne dann selektiv von dem nun bereits im wesentlichen rein vorliegenden Vorläuferprotein unter Erzeugung des erfindungsgemäßen Fusionsprotein abgespalten werden. Sofern die zusätzliche Domäne die Eignung des Fusionsproteins für die erfindungsgemäßen Assays nicht beeinflußt, besteht jedoch alternativ auch die Möglichkeit, auf den Abspaltungsschritt zu verzichten. Ein Beispiel für eine derartige Domäne besteht aus mehreren, z.B. 10, zusätzlich N-terminal angefügten Histidinresten ("His-tag"), die spezifisch an eine Metall-Chelat-Affinitätschromatographiesäule binden. Bezüglich aller genannten Techniken und den dafür erforderlichen Reagenzien, einschließlich Vektormolekülen, kann auf Standardliteratur (z.B. Sambrook, J., Fritsch, E.F., Maniatis, T. (1989), a.a.O.; Current Protocols in Molecular Biology (1991)) und eine unübersehbare Vielzahl von Einzelprotokollen verwiesen werden.

Ein weiterer zentraler Gegenstand der Erfindung sind Zellen, die eines oder mehrere der erfindungsgemäßen Fusionsproteine enthalten. Im Rahmen dieser Erfindung können dabei einzelne, mehrere oder auch alle Domänen des Fusionsproteins und/oder gegebenenfalls auch Teile einer oder mehrerer Domänen bezüglich der Wirts- oder Ausgangszelle heterolog sein.

Aufgrund der in dem Fusionsprotein enthaltenen Membranlokalisierungsdomäne liegt das Fusionsprotein in den Zellen membrangebunden vor. Auf diese Weise befinden sich die zweite und dritte Domäne des Fusionsproteins intrazellulär in unmittelbarer Nähe der Zellmembran.

Zur Herstellung der erfindungsgemäßen Zellen kann beispielsweise eine Transformation oder Transfektion von Ausgangszellen mit einem Expressionsvektor, der ein Gen für das erfindungsgemäße Fusionsprotein unter Kontrolle eines in der Ausgangszelle funktionsfähigen Promotors enthält, erfolgen. Als Ausgangszellen kommen prokaryotische wie eukaryotische Zellen in Frage. Beispiele für Ausgangszellen sind u.a. Bakterienzellen, wie solche der Gattung *Escherichia* oder *Bacillus*, beispielsweise bestimmte Stämme von *Escherichia coli* oder *Bacillus subtilis,* Hefezellen, wie bestimmte Stämme von *Saccharomyces cerevisiae*, Insektenzellen, tierische Zellen, wie COS-7, Vero, CHO-Zellen, Mäuse-Myelomzellen, humane FL-Zellen u.s.w.

Nach Transformation oder Transfektion einer Ausgangszelle kann das Gen für das erfindungsgemäße Fusionsprotein chromosomal, also im Chromosom integriert, oder als Bestandteil eines Episoms, insbesondere Plasmids, d.h extrachromosomal, in der transformierten oder transfizierten Zelle vorliegen. Gleiches gilt für eine zusätzliche Transformation oder Transfektion von Zellen auch mit anderen Genen, insbesondere Reportergenen oder -konstrukten, die, wie im Folgenden eingehender erläutert, im Rahmen besonderer Ausführungsformen der Erfindung vorgenommen wird.

Ein wesentliches Merkmal der erfindungsgemäßen Zellen besteht darin, daß bei fehlender Bindung oder, alternativ, bei Bindung von Ligand an die zweite Domäne des Fusionsproteins die dritte Domäne nicht in der Lage ist, die Aktivierung des sich an ein Ras-Protein anschließenden Signalwegs in den Zellen zu bewirken.

In einer bevorzugten Ausführungsform ist die dritte Domäne bei fehlender Bindung von Ligand an die zweite Domäne durch einen sich an das Fusionsprotein anlagernden, bevorzugt zelleigenen Multiproteinkomplex so komplexiert, daß die dritte Domäne nicht in der Lage ist, die Aktivierung des sich an ein Ras-Protein anschließenden Signalwegs in den Zellen zu bewirken. Bei Bindung von Ligand an die zweite Domäne erfolgt jedoch eine Konformationsänderung mit Auswirkungen auf die dritte Domäne, so daß in der Folge der Multiproteinkomplex zumindest teilweise von dem Fusionsprotein abdissoziiert und die dritte Domäne ihre Aktivität zur Aktivierung eines sich an ein Ras-Protein anschließenden Signalwegs in den Zellen ausüben kann.

In einer bevorzugten Ausführungsform dieser Erfindung ist die erfindungsgemäße Zelle dadurch gekennzeichnet, daß in Abwesenheit von Fusionsprotein zumindest unter bestimmten Bedingungen ein sich an ein Ras-Protein anschließender Signalweg in der Zelle nicht aktiviert werden kann, und insbesondere der Signalweg, zu dessen Aktivierung die dritte Domäne in der Lage ist. So sind im Stand der Technik Zellen bekannt, bei denen ein bestimmter ras-Signaltransduktionsweg temperaturabhängig aktiv bzw. inaktiv ist. Derartige Zellen können als Ausgangszellen für eine Expression des erfindungsgemäßen Fusionsproteins eingesetzt werden.

Die zumindest unter bestimmten Bedingungen vorliegende Inaktivierung eines ras-Signaltransduktionswegs resultiert aus einem zumindest unter den bestimmten Bedingungen nicht funktionsfähigen Ras-Protein und/oder Guaninnukleotid-Austauschfaktor. Die Inaktivierung kann auf Genmutation oder vollständiger oder teilweiser Gendeletion beruhen. Beispielsweise kann ein zelleigenes Ras-Protein inaktiviert werden, wenn dessen Membranlokalisierungssignal, in der Regel ein Farnesylierungssignal, deletiert wird. Gleiche Wirkung hätte eine Mutation in diesem Membranlokalisierungssignal, welche bewirkt, daß keine Bindung des Ras-Proteins an zelluläre Membranen mehr erfolgen kann. Ein Beispiel für eine Zelle mit einem temperaturabhängig defekten Guaninnukleotid-Austauschfaktor ist der *Saccharomyces cerevisiae-*Hefestamm cdc25-2. Bei diesem Stamm ist der Guaninnukleotid-Austauschfaktor bei einer restriktiven Temperatur von 33 bis 37°C, typischerweise 36°C, nicht mehr aktiv, jedoch bei einer Temperatur von beispielsweise 25°C voll funktionsfähig. Da der Guaninnukleotid-Austauschfaktor in diesem Hefestamm mit einem Ras-Protein zusammenwirkt, das einen auf den Zellzyklus einwirkenden und daher für das Zellwachstum essenziellen ras-Signaltransduktionsweg steuert, ist bei einer restriktiven Temperatur keine Vermehrung der Zellen des Hefestamms mehr festzustellen.

In analoger Weise zu Hefestämmen mit einer temperatursensitiven Mutation eines hefeeigenen SOS-Proteins (CDC25-2) kann auch ein Hefestamm mit einer temperatursenstiven Mutation eines hefeeigenen Ras-Proteins eingesetzt werden.

Alternativ kann für die Herstellung der erfindungsgemäßen Zellen aber auch eine Zelle, z.B. Hefezelle oder Säugetierzelle, verwendet werden, die zwar in der Lage ist, ein wildtypisches oder mutiertes, aber aktives CDC25/SOS-Protein oder Ras-Protein zu exprimieren, in der jedoch das dieses aktive CDC25/SOS-Protein oder Ras-Protein kodierende Gen unter der Kontrolle eines induzierbaren Promoters steht, durch welchen die Expression des Gens über die Wahl bestimmter Kulturbedingungen gezielt an- oder abgeschaltet werden kann. Beispiele für in diesem Zusammenhang einsetzbare induzierbare Promotoren sind der Galactose-Promotor oder Teile davon aus Hefe oder anderen Organismen. Dem Fachmann ist eine Vielzahl von hierzu geeigneten, induzierbaren Promotoren aus den unterschiedlichsten Organismen bekannt. Es können auch hybride Promotoren mit geeigneter Induzierbarkeit eingesetzt werden.

Exprimiert die erfindungsgemäße Zelle ein aktives CDC25/SOS-Protein oder ein aktives Ras-Protein, so kann in einer weiteren bevorzugten Ausführungsform der Erfindung das CDC25/SOS- oder Ras-Protein zusätzlich eine Modifizierung enthalten, durch die das Protein in der Zelle beschleunigt degradiert wird. Diese Modifizierung kann beispielsweise ein Ubiquitin-Signal oder ein sonstiges Signal sein, welches für den bevorzugten Abbau eines derartig modifizierten Proteins in der Zelle sorgt. Der Vorteil der Expression eines derartig modifizierten Proteins während einer Induktion des Promotors liegt darin, daß nach "Abschalten" des Promotors, d.h. nach Vorsehen von Kulturbedingungen, bei denen der Promotor nicht induziert ist und dementsprechend keine Transkription des CDC25/SOS- oder Ras-Gens mehr erfolgt, das noch zu Zeiten der Induktion des Promotors produzierte CDC25/SOS- oder Ras-Protein beschleunigt abgebaut wird. Dementsprechend wird bereits kurze Zeit nach "Abschalten" des Promotors kein derartiges aktives CDC25/SOS- oder Ras-Protein in der Zelle mehr nachzuweisen sein. In der bevorzugten Situation, wo die dritte Domäne des erfindungsgemäßen Fusionsproteins in der Lage ist, eben den durch das vorstehend angesprochene aktive CDC25/SOS- oder Ras-Protein aktivierten Signalweg zu aktivieren, ist es somit möglich, bereits kurze Zeit nach Wechsel der Kulturbedingungen zur Abschaltung des Promotors die Aktivierung dieses Signalwegs ausschließlich aufgrund der Wirkungen des erfindungsgemäßen Fusionsproteins zu messen, was einen beträchtlichen Zeitvorteil bedeuten kann. Zudem kann auf diese Weise das Hintergrundsignal aufgrund einer möglicherweise noch oder nach wie vor in geringem Umfang vorliegenden Aktivierung des Signalwegs, die nicht auf das erfindungsgemäße Fusionsprotein zurückzuführen ist, signifikant verringert werden.

Beruht die Inaktivierung oder Inaktivierbarkeit des zelleigenen ras-Signaltransduktionswegs auf einem Defekt oder Fehlen eines Guaninnukleotid-Austauschfaktors, so kann in dem bevorzugten Falle, wo das Fusionsprotein eine dritte Domäne aufweist, die eben diesen ras-Signaltransduktionsweg aktivieren kann, diese dritte Domäne die Aktivität eines funktionellen Guaninnukleotid-Austauschfaktors oder eines aktiven, insbesondere konstitutiv aktiven Ras-Proteins aufweisen, welcher bzw. welches den inaktiven ras-Signaltransduktionsweg aktivieren kann. Wird eine dritte Domäne mit einer Aktivität eines nicht konstitutiv aktiven Ras-Proteins eingesetzt, so wird sie sinnvollerweise die folgenden Eigenschaften aufweisen:
- sie benötigt eine Aktivierung durch einen andersartigen Guaninnukleotid-Austauschfaktor, der mit dem zelleigenen Ras-Protein des inaktiven ras-Signaltransduktionsweg nicht funktional wechselwirken kann. Gegebenenfalls kann dieser spezifisch geeignete Guaninnukleotid-Austauschfaktor als heterologer Faktor in der erfindungsgemäßen Zelle koexprimiert werden.

Ist die Inaktivierung oder Inaktivierbarkeit des zelleigenen ras-Signalwegs auf einen Defekt oder ein Fehlen eines zelleigenen Ras-Proteins zurückzuführen, so wird in dem bevorzugten Falle, wo das Fusionsprotein eine dritte Domäne aufweist, die eben diesen ras-Signalweg aktivieren kann, diese dritte Domäne die Aktivität eines aktiven, insbesondere konstitutiv aktiven Ras-Proteins aufweisen. Weist die dritte Domäne die Aktivität eines nicht-konstitutiv aktiven Ras-Proteins auf, so erfolgt deren Aktivierung bevorzugt durch einen zelleigenen Guaninnukleotid-Austauschfaktor, kann jedoch alternativ hierzu auch einen in der Zelle kozuexprimierenden heterologen Guaninnukleotid-Austauschfaktor benötigen.

Die für die Herstellung der erfindungsgemäße Zellen erforderlichen molekularbiologischen Techniken, z.B. Klonierung, Vektorkonstruktion, Transformation oder Transfektion, Selektionierung transformierter bzw. transfizierter Zellen und Züchtung der transformierten bzw. transfizierten Zellen u.s.w., sind dem Fachmann wohlbekannt und es existieren viele allgemeine Protokolle dafür, die gegebenenfalls allenfalls geringfügig angepaßt werden müssen, siehe z.B. Sambrook, J., Fritsch, E.F., Maniatis, T. (1989), a.a.O.; Current Protocols in Molecular Biology (1991) sowie zahlreiche Protokolle, die speziell für einen bestimmten Zelltyp erstellt wurden. Die Expression des Fusionsprotein kann dabei, wie erwähnt, ausgehend von einem innerhalb eines Episoms, beispielsweise Plasmides enthaltenen, extrachromosomal vorliegenden Gen wie auch ausgehend von einem in das Genom der Ausgangszelle integrierten Gen erfolgen. Für die Erzeugung von Zellen, bei denen ein bestimmter ras-Signaltransduktionsweg auf Ebene des Ras-Proteins oder eines Guaninnukleotid-Austauschfaktors inaktiviert ist, stehen dem Fachmann diverse Techniken zur gezielten Geninaktiyierung, beispielsweise durch "antisense"-Strategien, oder zur gezielten Einführung von Mutationen oder Deletionen in die jeweiligen Gene bzw. zugehörigen Genomabschnitte zur Verfügung. Insbesondere gibt es diverse bekannte Möglichkeiten zur Herstellung von Zellmutanten, in denen die Transkription der Gene z.B. für das Ras-Protein oder einen Guaninnukleotid-Austauschfaktor gezielt unter bestimmten Bedingungen, z.B. temperaturabhängig, inaktiviert werden kann. Hierzu enthalten diese Zellen diese Gene insbesondere in Verknüpfung mit Promotoren, die unter bestimmten Bedingungen, wie ab einer bestimmten Temperatur, inaktiv werden.

In einer besonderen Ausführungsform der Erfindung sind die erfindungsgemäßen Zellen auf einem festen Träger aufgebracht. Als geeignete Trägersubstanzen sind im Stand der Technik insbesondere Polysaccharide, z.B. Agarose, spezielle Kunststoffe, wie Polyacrylamide, Polystyrol, Polyvinylalkohol, Silikone, oder auch bestimmte Glasqualitäten bekannt. Der Träger kann hier in Form diskreter Teilchen, z.B. Kügelchen, oder als im wesentlichen plattenförmiges Substrat, z.B. in Form einer Mikrotiterplatte, vorliegen. Der Bedeckung des Trägers mit den Zellen kann vollständig sein, wie dies in der Regel z.B. bei Trägerkügelchen der Fall ist, oder auch nur auf Teilen oder Abschnitten desselben vorliegen, wie z.B. nur in den Vertiefungen einer Mikrotiterplatte. In einer bevorzugten Ausführungsform sind die erfindungsgemäßen Zellen auf sogenannten Biochips immobilisiert. Methoden zur Immobilisierung der Zellen auf diesen Trägern sind dem Fachmann bekannt. In Abhängigkeit von dem gewählten Trägertyp ist es möglich, daß die Zellen ohne weitere Maßnahmen an den Träger binden. In diesem Falle wird die feste Trägerphase mit einer im wesentlichen homogenen Population von Zellen inkubiert, wobei diese sich in der Folge an die feste Phase anheften. Alternativ kann die Immobilisierung beispielsweise auch mittels chemischer Reagenzien, wie Glutaraldehyd, Formalin u.s.w., erfolgen. Derartige Maßnahmen sind dem Fachmann bekannt.

Die erfindungsgemäßen Fusionsproteine und Zellen, die diese Fusionsproteine umfassen, sind die Grundlage mehrerer *in vivo-*Assayverfahren, die ebenfalls Gegenstand dieser Erfindung sind. Die im folgenden näher erläuterten Assayverfahren können bei Einsatz der ersten Variante, d.h. bei Aktivität der dritten Domäne *nur bei Ligandenbindung* an den Ligandenbindungsabschnitt (zweite Domäne) des erfindungsgemäßen Fusionsproteins u.a. dazu genutzt werden,
1. die Eignung einer Testsubstanz als Ligand für einen nukleären Rezeptor zu bestimmen, und in diesem Falle insbesondere Massenscreens mit Ligandenderivaten durchzuführen, um zu testen, welche Derivate in der Lage sind, an einen Ligandenbindungsabschnitt eines wildtypischen nukleären Rezeptors zu binden,
2. die Anwesenheit eines bestimmten Liganden in einer Probe zu detektieren,
3. die Konzentration eines solchen Liganden in einer Probe zu bestimmen,
4. nachzuweisen, ob eine Verbindung in der Lage ist, eine Bindungsaktivität eines Ligandenbindungsabschnitts eines nukleären Rezeptors gegenüber einem Liganden zu verändern, also als Agonist, Antagonist oder Inhibitor zu wirken, und in diesem Falle insbesondere Massenscreens zum Auffinden solcher Agonisten- oder Antagonistenverbindungen auszuführen; und
5. die Ligandenbindungsfunktion eines Polypeptids oder Proteins, bei denen eine solche Funktion vermutet wird, für Liganden von nukleären Rezeptoren nachzuweisen; bei den Polypeptiden oder Proteinen kann es sich insbesondere auch um durch Mutation von natürlichen Rezeptoren abgeleitete neuartige nukleäre Rezeptoren handeln, deren Ligandenbindungsfunktion noch bestätigt werden muß; in diesem Zusammenhang können insbesondere Massenscreens mit derartigen neuartigen, mutierten Ligandenbindungsabschnitten, die beispielsweise in Form einer Rezeptormutantenbibliothek, die insbesondere Rezeptormutanten mit zufallsbedingt lokalisierten Mutationen im Ligandenbindungsabschnitt enthält, durchgeführt werden, um neue künstliche, funktionelle Liganden-Rezeptor-Partner zu finden.

Ein erster Assay dient der Bestimmung der Eignung einer Testsubstanz als Ligand für einen Rezeptor- oder, synonym, Ligandenbindungsabschnitt eines nukleären Rezeptors und umfaßt die folgenden Schritte:
(a) Inkontaktbringen der Testsubstanz mit erfindungsgemäßen Zellen unter Bedingungen, bei denen in Abwesenheit des Fusionsproteins ein sich an ein Ras-Protein anschließender Signalweg in den Zellen nicht aktiviert werden kann, wobei das in den Zellen enthaltene Fusionsprotein eine zweite Domäne, die den genannten Rezeptorabschnitt umfaßt, und eine dritte Domäne, die in der Lage ist, den inaktiven, sich an ein Ras-Protein anschließenden Signalweg in den Zellen zu aktivieren, umfaßt,
(b) Untersuchten, ob eine Aktivierung des sich an ein Ras-Protein anschließenden Signalweges erfolgt ist.
Ein Nachweis der Aktivierung des sich an ein Ras-Protein anschließenden Signalweges zeigt dabei die Bindungsfähigkeit der Testsubstanz an die zweite Domäne des Fusionsproteins und dementsprechend an den Rezeptorabschnitt an.

Ein weiterer *in vivo*-Assay ermöglicht den Nachweis der Anwesenheit eines Liganden für einen Rezeptorabschnitt eines nukleären Rezeptors in einer Probe, die diesen möglicherweise enthält, und ist durch die folgenden Schritte gekennzeichnet
(a) Inkontaktbringen der Probe mit erfindungsgemäßen Zellen unter Bedingungen, bei denen in Abwesenheit von Fusionsprotein ein sich an ein Ras-Protein anschließender Signalweg in den Zellen nicht aktiviert werden kann, wobei das in den Zellen enthaltene Fusionsprotein eine zweite Domäne, die den genannten Rezeptorabschnitt umfaßt, und eine dritte Domäne, die in der Lage ist, den inaktiven, sich an ein Ras-Protein anschließenden Signalweg in den Zellen zu aktivieren, umfaßt,
(b) Untersuchen, ob eine Aktivierung des sich an ein Ras-Protein anschließenden Signalweges erfolgt ist.
Analog zu dem erstgenannten Assay zeigt ein Nachweis der Aktivierung des sich an ein Ras-Protein anschließenden Signalweges die Anwesenheit eines Liganden für die zweite Domäne des Fusionsproteins und dementsprechend für den Rezeptorabschnitt eines nukleären Rezeptors in der Probe an.

Als bevorzugte ras-Signalwege werden in diesem Zusammenhang, wie erläutert, Signalwege angesehen, die auf den Zellzyklus einwirken und deren Aktivierung für die Zellvermehrung essenziell ist. Alternative und ebenso bevorzugte ras-Signalwege dienen der Aktivierung von Transkriptionsfaktoren für Gene, die für die Zellvermehrung nicht essenziell sein müssen.

Der Nachweis der ras-Signalwegsaktivierung erfolgt bei den erfindungsgemäßen Assays bevorzugt indirekt, d.h. über phänotypische Änderungen, hier insbesondere Zellvermehrung oder Gen-bzw. Reportergenaktivität, in den Zellen.

Werden dementsprechend für die Assays Zellen eingesetzt, bei denen der inaktive, sich an ein Ras-Protein anschließende Signalweg ein Signalweg ist, der auf den Zellzyklus einwirkt und dessen Aktivierung für die Zellvermehrung essenziell ist, umfassen die vorstehend erläuterten Schritte (b), zu untersuchen, ob die Zellen unter den genannten Bedingungen vermehrungsfähig sind, wobei ein Nachweis der Vermehrungsfähigkeit der Zellen die Bindungsfähigkeit der Testsubstanz an bzw. die Anwesenheit eines Liganden für die zweite Domäne des Fusionsproteins und dementsprechend den Rezeptorabschnitt eines nukleären Rezeptors in der Probe anzeigt.

Setzt man für die Assays alternativ Zellen ein, bei denen der inaktive, sich an ein Ras-Protein anschließende Signalweg ein Signalweg ist, der auf die Aktivität eines Transkriptionsfaktors für ein Gen, das für die Zellvermehrung nicht notwendigerweise essenziell ist, einwirkt, so kann bei gleichzeitigem Vorliegen eines Konstrukts, umfassend eine Bindungsstelle für den Transkriptionsfaktor, einen damit zusammenwirkenden Minimalpromotor und ein unter Kontrolle des Minimalpromotors stehendes bezüglich der Assayzelle heterologes Reportergen, der Nachweis einer Expression des heterologen Reportergens für die Feststellung der Aktivierung des ras-Signaltransduktionswegs und damit einer erfolgten Ligandenbindung an die zweite Domäne herangezogen werden. Denn nur bei Aktivierung des ras-Signaltransduktionsweges kann es zu einer Aktivierung des genannten Transkriptionsfaktors kommen, welcher in der Folge über die Bindung an seine Bindungsstelle den Minimalpromotor aktivieren kann und damit die Expression des Reportergens ermöglicht.

Essenziell ist bei dieser Ausführungsform, daß es sich bei dem Reportergen und/oder dem dadurch kodierten Reporterprotein um ein bezüglich der Assayzelle heterologes Gen bzw. Protein handelt, dessen Anwesenheit spezifisch nur dann nachgewiesen werden kann, wenn die Expression des synthetischen Promotor-Reportergen-Konstrukts aufgrund der Aktivierung des spezifischen ras-Signalweges und der infolge auftretenden Aktivierung des spezifischen Transkriptionsfaktors erfolgt. Erfolgt der Nachweis nicht über einen direkten Nachweis des Transkriptions- oder Translationsprodukts mittels dafür spezifischer Nukleinsäuresonden oder Antikörper, sondern z.B. über die enzymatische Aktivität eines Translationsprodukts, muß bei einer Verwendung von Enzyme kodierenden Genen vorab sichergestellt werden, daß die verwendete Assayzelle vor der Transformation oder Transfektion mit dem synthetischen Promotor-Reportergen-Konstrukt eine enzymatische Aktivität, wie sie das bei Ligandenbindung exprimierte heterologe Enzym ausübt, nicht enthält. Entsprechendes gilt für die anderen Reporterproteintypen.

Alternativ kann als Reportergen auch ein bezüglich der Assayzelle homologes Gen eingesetzt werden. Eine Reportergenexpression infolge der Aktivierung eines Transkriptionsfaktors, die nur aufgrund der im Assay nachzuweisenden Aktivierung eines sich an ein Ras-Protein anschließenden Signalweges in den Zellen erfolgt, wird in diesem Falle zu einer Erhöhung der in den Zellen vorliegenden Mengen an Transkriptionsprodukt des Reportergens und gegebenenfalls auch einer erhöhten Menge an Tränslationsprodukt des Reportergens führen, welche anhand von Vergleichsversuchen ohne Einsatz von Ligand, z.B. mittels Northern-Blotting oder Western-Blotting, ermittelt werden können.

Bei Wahl dieser beiden letztgenannten Alternativen ist die Kenntnis des jeweiligen durch den gewählten ras-Signaltransduktionsweg aktivierten Transkriptionsfaktors sowie des mit diesem Transkriptionsfaktor zusammenwirkenden Promotorabschnitts bzw. von dessen Sequenz erforderlich. Um diese Assayvariante zu ermöglichen, wird die Assayzelle mit einem Konstrukt, umfassend den Promotor in funktionaler Verknüpfung mit dem Reportergen, transformiert bzw. transfiziert, was gegebenenfalls im Wege der Cotransformation oder Cotransfektion zusammen mit dem Konstrukt, das das Fusionsprotein kodierende Gen enthält, erfolgen kann. Wie erwähnt, können diese Konstrukte nach Transformation oder Transfektion einer Ausgangszelle chromosomal oder extrachromosomal, d.h. als Bestandteil eines Episoms, z.B. Plasmids, in der Assayzelle vorliegen.

Gegenwärtig sind bereits eine Reihe von ras-Signaltransduktionswegen z.B. in unterschiedlichen eukaryotischen Organismen vollständig auch bezüglich der dadurch aktivierten Transkriptionsfaktoren und den damit zusammenwirkenden Promotorbereichen erforscht. Somit steht dem Fachmann für eine diesbezügliche Auswahl eine Reihe von Möglichkeiten zur Verfügung.

In einer bevorzugten Ausführungsform der Erfindung umfaßt das Reporterprotein eine Modifizierung, durch die das Protein in der Zelle beschleunigt abgebaut oder degradiert wird. Diese Modifizierung kann beispielsweise ein Ubiquitinsignal oder sonstiges Signal sein, welches für den Abbau eines derartig modifizierten Proteins sorgt. Der Vorteil der Verwendung eines Reporterproteins, das in einer Assayzelle beschleunigt abgebaut wird, ist angesichts der Tatsache, daß unter den Assay-Bedingungen auch ohne Aktivierung des sich an ein Ras-Protein anschließenden Signalwegs durch das Fusionsprotein in der Assayzelle nahezu stets eine geringe Hintergrund-Expression des Reportergenkonstrukts nachzuweisen sein wird, offensichtlich: durch den beschleunigten Abbau des Reporterproteins wird das aus dieser Hintergrund-Expression resultierende Signal bei Detektion auf Proteinebene, d.h. des Reporterproteins, signifikant verringert, da es zu keiner Akkumulation von Reporterprotein über die zeit kommt. Wird jedoch die Expression des Reporterproteins durch Ligandenbindung an das Fusionsprotein und daraus resultierende Aktivierung eines sich an ein Ras-Protein anschließenden Signalwegs spezifisch aktiviert, kann aufgrund des geringen Hintergrundsignals eine eindeutige Detektion erfolgen. Da die Halbwertszeit des Reporterproteins in der Assayzelle stets ausreichend lang sein wird, wird der Nachweis des infolge der Ligandenbindung an das Fusionsprotein produzierten Reporterproteins durch den beschleunigten Abbau desselben in keiner Weise beeinträchtigt werden.

Die für die Herstellung von Transformations- oder Expressionsvektoren, die das Reportergen in funktionaler Verknüpfung mit einem geeigneten spezifischen Promotor enthalten, sowie die Transformation oder Transfektion von Zellen erforderlichen molekularbiologischen Techniken, z.B. Klonierung, Vektorkonstruktion u.s.w., sind dem Fachmann wohlbekannt und es existieren zahlreiche allgemeine Protokolle dafür, die gegebenenfalls allenfalls einer geringfügigen Anpassung bedürfen (siehe z.B. Sambrook, J., Fritsch, E.F., Maniatis, T. (1989), a.a.O.; Current Protocols in Molecular Biology (1991)). Auch die Ergänzung bzw. Fusion eines Reportergens mit einem Sequenzabschnitt, der einen Signalabschnitt kodiert, welcher einen beschleunigten Abbau des exprimierten Reporterproteins bewirkt, z.B. ein Ubiquitin-Signal, liegt ohne weiteres im Vermögen eines Fachmanns.

Als hier einsetzbare Reportergene sind dem Fachmann zahlreiche Gene bekannt, die Proteine kodieren, die einem einfachen und schnellen Nachweis zugänglich sind. Beispiele hierfür sind Gene, die enzymatisch aktive Proteine, z.B. β-Galactosidase, fluoreszierende Proteine, z.B. GFP ("green fluorescence protein") oder chemolumineszierende Proteine kodieren. Eine weitere Möglichkeit besteht in Genen, die Proteine kodieren, die mittels spezifischer Antikörper nachgewiesen werden können. Hierbei trägt dann der Antikörper eine nachweisbare Markierung oder kann seinerseits durch einen sekundären, markierten Antikörper nachgewiesen werden. Derartige Möglichkeiten sind im Stand der Technik wohlbekannt. Wie bereits vorstehend erläutert, ist neben dem Erfordernis der Nachweisbarkeit allein essenziell, daß das in der Zelle nachzuweisende Ereignis, z.B. enzymatische Aktivität, Antikörperbindung, Fluoreszenz, Chemolumineszenz, in Abwesenheit des Konstrukts mit dem Gen für das Reporterprotein nicht nachweisbar ist.

Alternativ kann die Transkription des Reportergens anhand der gebildeten mRNA mittels dafür spezifischer Sonden durch Northern-Blotting nachgewiesen werden.

Ein weiterer *in vivo*-Assay erlaubt die quantitative Bestimmung der Konzentration eines Liganden für den Rezeptorabschnitt eines nukleären Rezeptors in einer Probe, die diesen enthält, und umfaßt die folgenden Schritte:
(a) Inkontaktbringen eines Aliquots der Probe mit erfindungsgemäßen Zellen unter Bedingungen, bei denen in Abwesenheit des Fusionsproteins ein sich an ein Ras-Protein anschließender Signalweg in den Zellen nicht aktiviert werden kann, wobei das in den Zellen enthaltene Fusionsprotein eine zweite Domäne, die den genannten Rezeptorabschnitt umfaßt, und eine dritte Domäne, die in der Lage ist, den inaktiven, sich an ein Ras-Protein anschließenden Signalweg in den Zellen zu aktivieren, umfaßt,
(b) quantitatives Nachweisen des Ausmaßes der Aktivierung des sich an ein Ras-Protein anschließenden Signalweges auf direktem oder indirektem Weg und
(c) Ermittlung der Konzentration des Liganden in der Probe durch Vergleich des ermittelten Aktivierungsausmaßes mit entsprechenden Werten, die für bekannte Standardkonzentrationen des Liganden ermittelt wurden.

Verwendet man für den quantitativen Nachweis des Schrittes (b) Zellen, bei denen der zumindest unter bestimmten Bedingungen inaktive ras-Signaltransduktionsweg ein Signalweg ist, der auf den Zellzyklus einwirkt und dessen Aktivierung für die Zellvermehrung essenziell ist, erfolgt dieser auf einfache Weise, indem die Vermehrung der Zellen zu einem festgelegten Zeitpunkt oder die Vermehrungsrate der Zellen unter den genannten Bedingungen bestimmt wird. Die erhaltenen Daten werden dann mit anhand von Standardpräparationen bekannter Konzentration erhaltenen Daten verglichen und die Konzentration der Probe rechnerisch ermittelt.

Alternativ kann auch hier der quantitative Nachweis des Ausmaßes der ras-Signalwegsaktivierung anhand des Ausmaßes der Expression eines Reportergens in der Zelle erfolgen. Wie vorstehend erläutert, kann dessen Expression nur aufgrund der infolge der Aktivierung des sich an ein Ras-Protein anschließenden Signalwegs bewirkten Aktivierung eines spezifischen Transkriptionsfaktors erfolgen. Bevorzugt erfolgt der Assay bei dieser Nachweisvariante unter Bedingungen, die eine Zellvermehrung ausschließen, beispielsweise durch Verwendung der cdc25-2-Hefemutante bei restriktiven Temperaturen, so daß die Menge an Transkriptionsprodukt des Reportergens oder die exprimierte Menge an Reporterprotein zu einem bestimmten Zeitpunkt oder alternativ die Expressionsrate dieses Reportergens bezogen auf das Transkriptions- oder Translationsprodukt von diesem bei im wesentlichen gleichbleibender Zellzahl bestimmt werden kann. Jedoch kann die quantitative Bestimmung auch unter Proliferationsbedingungen erfolgen, wenn gleichzeitig fortlaufend oder in bestimmten Zeitabständen die Zellzahl bestimmt wird und die ermittelten Werte für die Reportergenexpression in Werte pro Einheitswert der Zellzahl umgerechnet werden.

Alternativ kann auch hier ein Nachweis über die Expression eines zu der Wirtszelle homologen Reportergens eingesetzt werden, wobei hier die jeweils beobachtbare Zunahme der Expression des Reportergens gegenüber dem in Zellen ohne Aktivierung des ras-Signalwegs vorliegenden Expressionsniveau für die Ermittlung des Ergebnisses herangezogen wird.

Ein weiterer alternativer *in vivo*-Assay ermöglicht den Nachweis, ob eine Verbindung in der Lage ist, eine Bindungsaktivität eines Rezeptorabschnitts eines nukleären Rezeptors gegenüber einem Liganden zu verändern, also als Agonist, Antagonist oder Inhibitor zu wirken. Dieser Assay ist durch die folgenden Schritte gekennzeichnet:
(a) Inkontaktbringen des Liganden in Anwesenheit der Verbindung mit erfindungsgemäßen Zellen unter Bedingungen, bei denen die Verbindung in die Zellen diffundieren kann oder sie von den Zellen produziert wird und bei denen in Abwesenheit von Fusionsprotein ein sich an ein Ras-Protein anschließender Signalweg in den Zellen nicht aktiviert werden kann, wobei das in den Zellen enthaltene Fusionsprotein eine zweite Domäne, die den genannten Rezeptorabschnitt umfaßt, und eine dritte Domäne, die in der Lage ist, den inaktiven, sich an ein Ras-Protein anschließenden Signalweg in den Zellen zu aktivieren, umfaßt,
(b) Untersuchen, ob und gegebenenfalls in welchem Ausmaß eine Aktivierung des sich an ein Ras-Protein anschließenden Signalweges erfolgt ist,
(c) Vergleichen des Untersuchungsergebnisses von Schritt (b) mit einem Untersuchungsergebnis, das bei Ausführen des Assay in Abwesenheit der Verbindung erhalten wird.
Dabei zeigt eine verstärkte Aktivierung des ras-Signaltransduktionsweges in Anwesenheit der Verbindung eine Agonistenfunktion dieser Verbindung, eine verringerte oder gegebenenfalls auch vollständig fehlende Aktivierung dagegen eine Antagonisten- oder Inhibitorfunktion der Verbindung an.

Schritt (a) kann dabei umfassen, die Verbindung vor dem Liganden den Zellen zuzusetzen, wobei gegebenenfalls eine Vorinkubation der Verbindung mit den Zellen vorgenommen werden kann, die Verbindung separat, aber gleichzeitig mit dem Liganden den Assayzellen zuzusetzen oder die Verbindung vorab mit dem Liganden zu mischen und gegebenenfalls eine Vorinkubation der beiden Verbindungen durchzuführen und erst dann die Mischung den Assayzellen zuzusetzen.

Wird die Verbindung den Zellen zugesetzt, muß sichergestellt sein, daß die Verbindung auch in die Zellen diffundieren kann, um dort mit dem Fusionsprotein wechselzuwirken. Handelt es sich bei der Verbindung um ein Peptid, Polypeptid oder Protein, so kann die Verbindung auch durch Expression eines dafür kodiertenden Gens innerhalb der Zelle selbst hergestellt werden. Zu diesem Zweck können die Zellen mit einem Expressionsvektor, der ein solches Gen enthält, transformiert oder transfiziert werden. Die hierfür erforderlichen Mittel und Methoden sind dem Fachmann wohlbekannt.

Wird die auf ihre Agonisten- oder Antagonistenwirkung zu testende Verbindung in der Assayzelle exprimiert, so erfolgt die Expression des dafür kodierenden Gens vorzugsweise unter Kontrolle eines konstitutiv aktiven Promotors sowie unter Verwendung von Zellen, in denen der ras-Signaltransduktionsweg nur unter den speziellen Assaybedingungen inaktiviert wird. Ein solches System ermöglicht es, auszuschließen, daß allein die Expression der Verbindung in der Zelle Veränderungen hervorruft, die das Ergebnis des Assay verfälschen könnten. Für diesen Ausschluß ist erforderlich, die Aktivität des unter restriktiven Bedingungen inaktivierten zelleigenen ras-Signaltransduktionsweges unter nicht-restriktiven Bedingungen und in Abwesenheit von Ligand nachzuweisen. Wird unter nicht-restriktiven Bedingungen und in Abwesenheit von Ligand gearbeitet, ist das Fusionsprotein inaktiv, so daß die nachweisbare Aktivierung des ras-Signaltransduktionsweges anzeigt, daß das Expressionsprodukt mit keiner Komponente des ras-Signaltransduktionswegs, und insbesondere nicht mit dem dafür spezifischen Ras-Protein oder Guaninnukleotid-Austauschfaktor, interferiert. Auf diese Weise kann ausgeschlossen oder die Wahrscheinlichkeit minimiert werden, daß das Expressionsprodukt anstatt mit der zweiten Domäne mit der dritten Domäne wechselwirkt, so daß deren Fähigkeit zur Aktivierung des ras-Signaltransduktionsweges beseitigt wird.

Ein entsprechender Test auf Aktivierung des jeweiligen ras-Signaltransduktionswegs unter nicht-restriktiven Bedingungen, in Anwesenheit der Verbindung und in Abwesenheit von Ligand wird analog bei einer Verbindung, die den Assayzellen von außen zugesetzt wird, vorgenommen.

Handelt es sich bei dem unter den Assaybedingungen inaktivierbaren ras-Signaltransduktionsweg um einen, dessen Aktivierung für die Zellvermehrung essenziell ist, so wird unter nicht-restriktiven Bedingungen einfach die normale Vermehrbarkeit der Zellen sichergestellt. Erfolgt der Nachweis unter Nutzung einer Reportergenaktivität, so ist ein Nachweis dieser Reportergenaktivität unter nicht-restriktiven Bedingungen erforderlich.

Für den Nachweis unter den restriktiven Bedingungen des Assay in Schritt (b) besteht ebenfalls beispielsweise die Möglichkeit, die Aktivierung des sich an ein Ras-Protein anschließenden Signalweges über die gegebenenfalls erfolgende Expression eines zu den Zellen heterologen Reportergens, die nur aufgrund der infolge der Aktivierung des sich an ein Ras-Protein anschließenden Signalweges erfolgenden Aktivierung eines spezifischen Transkriptionsfaktors erfolgt, nachzuweisen. Der bei Nachweis von Aktivierung des sich an ein Ras-Protein anschließenden Signalweges erfolgende Nachweis des Ausmaßes dieser Aktivierung, kann eine quantitative Bestimmung umfassen, bei welcher die in den Zellen vorliegende Menge an Transkriptions- oder Translationsprodukt (Reporterprotein) des Reportergens zu einem bestimmten Zeitpunkt oder die Transkriptionsrate des Reportergens oder die Expressionsrate des Reporterproteins unter den genannten Bedingungen bestimmt wird.

Alternativ kann auch nur eine Analyse von Aliquots, d.h. gleichen Volumina der bis auf den Zusatz der Verbindung identisch erzeugten und behandelten Assaylösungen, bevorzugt unter Sicherstellen gleicher oder im wesentlichen gleicher Zellzahlen in diesen Aliquots, vorgenommen werden. In diesem Falle erfolgt keine absolute Quantifizierung des Expressionsniveaus des Reportergens, sondern es wird nur ein relativer Vergleich der Expressionsniveaus in beiden Assays ermöglicht.

In dem Falle, wo der Vergleich in Schritt (c) ergibt, daß in Anwesenheit der Verbindung eine stärkere Expression des Reportergens auftritt; ist eine Agonistenwirkung der Verbindung und in dem Falle, wo der Vergleich in Schritt (c) ergibt, daß in Anwesenheit der Verbindung eine geringere Expression des Reportergens auftritt, eine Antagonistenwirkung der Verbindung anzunehmen. Wie der vorstehend beschriebene quantitative Assay wird auch dieser Assay bevorzugt unter Bedingungen durchgeführt wird, bei denen keine Vermehrung der Zellen auftritt.

Alternativ kann das für den Nachweis eingesetzte Reportergen auch hier zu der Assayzelle homolog sein, wobei hier die jeweils beobachtbare Zunahme der Expression, d.h. Transkription und/oder Translation, des Reportergens gegenüber dem in Zellen ohne Aktivierung des ras-Signalwegs vorliegenden Expressionsniveau für die Ermittlung des Ergebnisses herangezogen wird.

Werden für den Assay Zellen eingesetzt, bei denen der inaktive, sich an ein Ras-Protein anschließende Signalweg ein Signalweg ist, der auf den Zellzyklus einwirkt und dessen Aktivierung für die Zellvermehrung essenziell ist, umfaßt Schritt (b), zu untersuchen, ob und gegebenenfalls in welchem Ausmaß die Zellen unter den genannten Bedingungen vermehrungsfähig sind. In dem Falle, wo der Vergleich in Schritt (c) ergibt, daß in Anwesenheit der Verbindung eine stärkere Zellvermehrung auftritt, ist auf eine Agonistenwirkung der Verbindung und in dem Falle, wo der Vergleich in Schritt (c) ergibt, daß in Anwesenheit der Verbindung eine geringere Zellvermehrung auftritt, auf eine Antagonistenwirkung der Verbindung zu schließen.

Wie sämtliche Assays im Rahmen dieser Erfindung eignet sich auch dieser Assay insbesondere für ein Massenscreening, hier auf Agonisten- und Antagonistenverbindungen für nukleäre Rezeptoren.

Alternativ kann dieser Assay auch als zusätzlicher Test zur Bestätigung der Ligandenbindungseigenschaft eines neuen, insbesondere synthetischen Ligandenbindungsabschnitts oder zur Bestätigung der Eignung einer Testsubstanz als Ligand für den Ligandenbindungsabschnitt herangezogen werden. Liegt eine Ligandenbindungseigenschaft des Ligandenbindungsabschnitts vor bzw. liegt eine Eignung als Ligand für den Ligandenbindungsabschnitt vor, so müßte bei Verwendung eines bekannten Agonisten für den zum ersten Nachweis der Ligandeneigenschaft eingesetzten Liganden bzw. für den Ligandenbindungsabschnitt eine verstärkte Aktivierung des Ras- oder Ras-ähnlichen Signalweges beobachtet werden, und bei Verwendung eines bekannten Antagonisten für den Liganden bzw. den Ligandenbindungsabschnitt im Gegenzug eine verringerte Aktivierung des Ras- oder Ras-ähnlichen Signalweges.

Ein weiterer alternativer *in vivo*-Assay ermöglicht den Nachweis, ob ein Polypeptid oder Protein, bei dem eine Ligandenbindungsfunktion eines nukleären Rezeptors vermutet wird, diese auch tatsächlich aufweist. Dieser Assay umfaßt die folgenden Schritte:
(a) Inkontaktbringen von erfindungsgemäßen Zellen mit dem Liganden unter Bedingungen, bei denen in Abwesenheit des Fusionsproteins ein sich an ein Ras-Protein anschließender Signalweg in den Zellen nicht aktiviert werden kann, wobei das in den Zellen enthaltene Fusionsprotein eine zweite Domäne, die das zu untersuchenden Polypeptid oder Protein umfaßt, und eine dritte Domäne, die in der Lage ist, den inaktiven, sich an ein Ras-Protein anschließenden Signalweg in den Zellen zu aktivieren, umfaßt,
(b) Untersuchen, ob eine Aktivierung des sich an ein Ras-Protein anschließenden Signalweges erfolgt ist.
Ein Nachweis der Aktivierung des sich an ein Ras-Protein anschließenden Signalweges zeigt an, daß die zweite Domäne des Fusionsproteins und dementsprechend das zu untersuchende Polypeptid oder Protein eine Ligandenbindungsfunktion eines nukleären Rezeptors aufweist.

Beispielsweise kann das in den Zellen enthaltene Fusionsprotein eine zweite Domäne umfassen, die einen von einem natürlich vorkommenden Rezeptorabschnitt eines nukleären Rezeptors durch Mutation abgeleiteten Rezeptorabschnitt enthält.

Wie zuvor, kann die Aktivierung des ras-Signaltransduktionsweges bei Verwendung von Zellen, bei denen der zumindest unter bestimmten Bedingungen inaktive ras-Signaltransduktionsweg ein Signalweg ist, der auf den Zellzyklus einwirkt und dessen Aktivierung für die Zellvermehrung essenziell ist, anhand einer gegebenenfalls erfolgenden Zellvermehrung nachgewiesen werden.

Alternativ kann der Nachweis der Aktivierung des ras-Signaltransduktionsweges auch anhand der gegebenenfalls feststellbaren Expression eines Reportergens in den Zellen erfolgen. Wie erläutert, erfolgt dessen Expression, wenn es sich um ein heterologes Reportergen handelt, nur aufgrund der infolge der Aktivierung des sich an ein Ras-Protein anschließenden Signalweges erfolgenden Aktivierung eines spezifischen Transkriptiönsfaktors. Im Falle eines homologen Reportergens wird auf den Nachweis der Erhöhung der Reportergenexpression, z.B. aufgrund höherer Mengen an Transkriptions- oder Translationsprodukt, im Vergleich zu dem Expressionsniveau ohne Aktivierung des spezifischen Ras-Signaltransduktionswegs abgestellt.

Bei Einsatz der zweiten Variante, d.h. Aktivität der dritten Domäne nur bei *fehlender* Ligandenbindung an den Ligandenbindungsabschnitt (zweite Domäne) des erfindungsgemäßen Fusionsproteins, können erfindungsgemäße Assayverfahren u.a. dazu genutzt werden,
1. die Eignung einer Testsubstanz als Ligand für einen nukleären Rezeptor zu bestimmen, und in diesem Falle insbesondere Massenscreens mit Ligandenderivaten durchzuführen, um zu testen, welche Derivate in der Lage sind, an den Ligandenbindungsabschnitt eines wildtypischen nukleären Rezeptors zu binden,
2. die Anwesenheit eines bestimmten Liganden in einer Probe zu detektieren,
3. nachzuweisen, ob eine Verbindung in der Lage ist, eine Bindungsaktivität eines Ligandenbindungsabschnitts eines nukleären Rezeptors gegenüber einem Liganden zu verändern, also als Agonist, Antagonist oder Inhibitor zu wirken, und in diesem Falle insbesondere Massenscreens zum Auffinden solcher Agonisten- oder Antagonistenverbindungen auszuführen; und
4. die Ligandenbindungsfunktion eines Polypeptids oder Proteins, bei denen eine solche Funktion vermutet wird, für Liganden bestimmter nukleärer Rezeptoren nachzuweisen; auch hier kann es sich bei den Polypeptiden oder Proteinen insbesondere um durch Mutation von natürlichen Rezeptoren abgeleitete neuartige Ligandenbindungsabschnitte von Rezeptoren handeln, deren Ligandenbindungsfunktion noch bestätigt werden muß; in diesem Zusammenhang können insbesondere Massenscreens mit derartigen neuartigen, mutierten Ligandenbindungsabschnitten, die beispielsweise in Form einer Rezeptormutantenbibliothek, die insbesondere Rezeptormutanten mit zufallsbedingt lokalisierten Mutationen im Ligandenbindungsabschnitt enthält, durchgeführt werden, um neue künstliche, funktionelle Liganden-Rezeptor-Partner zu finden.

Die vorstehend genannten Assays können im wesentlichen analog zu den im Vorangehenden erläuterten Assayvarianten unter Nutzung der ersten Variante erfolgen, wobei jedoch in diesem Falle der Nachweis der Aktivierung des Ras- oder Ras-ähnlichen Signalwegs die Abwesenheit eines Liganden für den Ligandenbindungsabschnitt des untersuchten Fusionsproteins in der Assayzelle anzeigt. Die letztgenannten Assays werden demzufolge in der Regel zwei Nachweise umfassen, und zwar einen Nachweis in Anwesenheit von (potentiellem) Ligand, wobei bei Ligandenbindungseigenschaft des (potentiellen) Liganden für den Ligandenbindungsabschnitt (zweite Domäne) des erfindungsgemäßen Fusionsproteins keine Aktivierung des Ras- oder Ras-ähnlichen Signalwegs nachzuweisen sein wird, sowie einen weiteren Nachweis in Abwesenheit des (potentiellen) Liganden, wobei eine derartige Aktivierung regelmäßig nachgewiesen werden wird.

Bei dem Nachweis, ob eine Verbindung in der Lage ist, eine Bindungsaktivität eines Ligandenbindungsabschnitts eines nukleären Rezeptors gegenüber einem Liganden zu verändern, also als Agonist, Antagonist oder Inhibitor zu wirken, erfolgt der Nachweis bei gleichzeitiger Anwesenheit von Ligand und Verbindung insbesondere über
a) die bei Antagonisten- oder Inhibitorwirkung der Verbindung möglicherweise doch nachweisbare Aktivierung des Ras- oder Ras-ähnlichen Signalwegs, beispielsweise mit anschließender Bestimmung der für eine vollständige Inaktivität des Ras- oder Ras-ähnlichen Signalwegs erforderlichen Ligandenkonzentration bei einer bestimmten Konzentration an Verbindung oder einer Bestimmung der Abhängigkeit der Signalwegs-Aktivierung von der Konzentration an Verbindung bei einer bestimmten Ligandenkonzentration; oder
b) die bei Agonistenwirkung der Verbindung bereits bei einer geringeren Ligandenkonzentration vollständigen Inaktivität des Ras- oder Ras-ähnlichen Signalwegs; auch hier kann die Abhängigkeit der vollständigen Inaktivität des Ras- oder Ras-ähnlichen Signalwegs von der Konzentration der Verbindung und/oder des Liganden im Einzelnen bestimmt werden.

Bei dem Nachweis einer Ligandenbindurigsfunktion eines Polypeptids oder Proteins, bei denen eine solche Funktion vermutet wird, für Liganden bestimmter Rezeptoren erfolgt der Nachweis der Ligandenbindungsfunktion analog über die Inaktivität des Ras- oder Ras-ähnlichen Signalwegs in Anwesenheit von Ligand bei Aktivität dieses Signalwegs in Abwesenheit von Ligand.

Die Detektion von nukleärer Rezeptor-Liganden-Interaktionen mittels der erfindungsgemäßen Assayverfahren, Zellen und Fusionsproteine ist nicht auf eukaryotische Zellen als *in vivo-*Testsystem beschränkt, sondern kann wahlweise auch in prokaryotischen Zellen erfolgen.

Bezüglich der Assaybedingüngen sind keine bestimmten allgemeinen Vorgaben erforderlich. Im Falle eines Nachweises der gegebenenfalls erfolgenden Zellvermehrung ist jedoch darauf zu achten, daß das verwendete Medium eine solche grundsätzlich ermöglicht. Sofern in den Zellen Gene und/oder Promotoren, wie erläutert, durch Wahl bestimmter Assaybedingungen inaktiviert werden können und im Zuge des Assays auch inaktiviert werden sollen, sind diese Bedingungen, wie z.B. eine bestimmte restriktive Assaytemperatur, bei cdc25-2-Zellen z.B. 33 - 37°C, während des Assay einzuhalten. Das gewählte Reaktionsmedium sollte ferner nicht mit der Testverbindung oder dem Liganden, die diesem zugesetzt werden, auf eine Weise wechselwirken, daß der Assay beeinträchtigt wird.

Als Liganden können in sämtlichen vorstehend erläuterten Assayverfahren natürliche vorkommende Substanzen, wie Hormone, insbesondere Steroidhormone, Vitamine, z.B. Vitamin D, Thyroxin oder Retinsäure, wie auch in der Natur nicht vorkommende Substanzen, z.B. synthetische Derivate natürlicher Liganden oder Giftstoffe, wie Dioxin, untersucht bzw. bestimmt werden. Da die Liganden nukleärer Rezeptoren überwiegend kleine Moleküle mit geringer relativer Molekülmasse und von überwiegend hydrophober Natur darstellen, diffundieren diese ohne weitere Maßnahmen in die erfindungsgemäßen Assayzellen, um dort mit dem intrazellulär und unmittelbar an der Zellmembran lokalisierten Ligandenbindungsabschnitt des Fusionsproteins eine Bindung einzugehen. Sofern gewünscht und/oder erforderlich, können die Zellen jedoch vor dem Assay in geeigneter Weise vorbehandelt werden, um die äußere Zellmembran für den Durchtritt der Testverbindung oder des Liganden durchlässiger zu machen. Ein Beispiel hierfür ist die Herstellung von zellwandlosen Zell-"ghosts" durch z.B. enzymatische Behandlung der Zellen, beispielsweise von zellwandlosen Hefezellen. Derartige wie auch auf andere Weise hergestellte Zell-"ghosts" sowie Zellen mit zur Erhöhung der Permeabilität anderweitig modifizierter Zellwand werden im vorliegenden Zusammenhang von dem Begriff "Zellen" mit umfaßt.

Sollte es sich bei den zu testenden Liganden um Peptide, Polypeptide oder Proteine handeln, so können diese auch in der Assayzelle ausgehend von in die Assayzelle eingeschleusten Nukleinsäurekonstrukten, die diese kodieren, exprimiert werden, in einer speziellen Variante auch nicht konstitutiv, sondern unter Kontrolle eines induzierbaren Promotors; dabei können die Konstrukte nach Einschleusen in die Assayzelle chromosomal oder extrachromosomal, d.h. als Bestandteil eines Episoms, z.B. Plasmids, in dieser vorliegen. Das Inkontaktbringen der Assayzelle mit dem Liganden erfolgt bei nicht-konstitutiver Expression dementsprechend unter den Bedingungen, bei denen die Expression des zu testenden Liganden in der Zelle induziert wird. Dem Fachmann sind zu diesem Zweck eine Vielzahl von induzierbaren Promotoren, die beispielsweise durch bestimmte Temperaturen oder chemische Verbindungen induzierbar sind, bekannt.

Allgemein ist festzuhalten, daß bei Hinzusetzen des zu testenden Liganden zu der Assayzelle von außen alle in dem erfindungsgemäßen Assaysystem zusammenwirkenden Komponenten, d.h. insbesondere das Fusionsprotein sowie jegliche Komponenten des spezifisch durch die dritte Domäne des Fusionsproteins aktivierten, sich an ein Ras-Protein anschließenden Signalwegs, die nur an diesem spezifischen Signalweg beteiligt sind, in der Assayzelle konstitutiv exprimiert werden können. Bei einer Expression des zu testenden Liganden in der Assayzelle können bei der ersten Variante, in welcher die dritte Domäne ihre Aktivität zur Aktivierung eines sich an ein Ras-Protein anschließenden Signalwegs nur bei Bindung von Ligand an den Ligandenbindungsabschnitt des untersuchten Fusionsproteins ausüben kann, alle Komponenten des Assaysystems, nun einschließlich des Liganden, bis auf eine in der Assayzelle konstitutiv exprimiert werden. Die Assaysystem-Komponente(n), deren Gen(e) unter Kontrolle eines induzierbaren Promotors vorgesehen wird bzw. werden, wird bzw. werden dann insbesondere nur unter Assaybedingungen, d.h. bei Untersuchung der möglicherweise erfolgten Aktivierung des Ras- oder Ras-ähnlichen Signaltransduktionsweges, aufgrund der gezielten Induktion des/der jeweils eingesetzten induzierbaren Promotors bzw. Promotoren exprimiert. Bei der zweiten Variante, bei welcher die dritte Domäne des Fusionsproteins nur bei fehlender Ligandenbindung an die zweite Domäne des Fusionsproteins ihre Aktivität ausüben kann, wird bei Expression des Liganden in der Assayzelle dessen Gen stets unter Kontrolle eines induzierbaren Promotors vorgesehen werden, um einen Nachweis auch in Abwesenheit des Liganden zu ermöglichen.

Der Nachweis der Aktivierung des ras-Signaltransduktionswegs erfolgt je nach Nachweisstrategie auf dem Fachmann geläufige Weise. Befinden sich die Zellen während des Assay immobilisiert an einem festen Träger, so kann es insbesondere bei Nachweis einer Reportergenaktivität oder -transkription oder eines Reporterproteins erforderlich oder hilfreich sein, die Zellen vor der Nachweisreaktion zu solubilisieren, d.h. sie von dem Träger abzulösen und ggf. auch aufzubrechen. Auch die hierfür erforderlichen Maßnahmen und Reagenzien sind dem Fachmann wohlbekannt.

Die Erfindung stellt darüber hinaus Kits zur Verwendung in den erfindungsgemäßen Assays bereit, welche es z.B. ermöglichen, schnell und effizient zu bestimmen, ob ein spezifischer Ligand in der Lage ist, an einen bestimmten nukleären Rezeptor oder Teile von solchen zu binden.

Ein erster Kit der Erfindung zur Verwendung in den Assayverfahren zur Bestimmung der Eignung einer Testsubstanz als Ligand für einen Rezeptorabschnitt eines nukleären Rezeptors, zur Bestimmung der Anwesenheit eines Liganden für einen Rezeptorabschnitt eines nukleären Rezeptors in einer Probe, zur Konzentrationsbestimmung eines solchen Liganden sowie zur Charakterisierung von Verbindungen als mögliche Agonisten oder Antagonisten bezüglich nukleärer Rezeptor-Liganden-Wechselwirkungen umfaßt jeweils erfindungsgemäße Zellen mit den vorstehend bei den Assayverfahren im Einzelnen erläuterten Eigenschaften. So enthalten die Zellen des Kits beispielsweise bei einem vorgesehenen Nachweis einer Reportergenaktivität zusätzlich ein Konstrukt mit einer Bindungsstelle für den Transkriptionsfaktor, der spezifisch durch den ras-Signalweg, dessen Aktivierung mittels der Assays nachgewiesen werden soll, aktiviert wird, einem Minimalpromotor und dem Reportergen. Alternativ kann der Kit wie auch alle folgenden einen Transformations- bzw. Transfektionsvektor, der das Konstrukt enthält, umfassen. Auf diese Weise kann der Verwender des Kits bei Wahl dieses Nachweisweges die im Kit enthaltenen Assayzellen durch Transformation oder Transfektion mit diesem Konstrukt ausstatten. In einer weiteren Ausführungsform dieses und aller folgenden Kits enthält der separat im Kit bereitgestellte Transformations- bzw. Transfektionsvektor nur die Bindungsstelle für den Transkriptionsfaktor und den damit funktional verknüpften Minimalpromotor sowie eine geeignet vorgesehene Insertionsstelle für die Insertion eines durch den Verwender frei wählbaren Reportergens.

Darüber hinaus kann dieser Kit wie auch alle folgenden unter anderem gegebenenfalls auch einen Assaypuffer, Reagenzien zur Detektion der phänotypischen Aktivierung des sich an ein Ras-Protein anschließenden Signaltransduktionsweges in diesen Zellen und/oder eine Gebrauchsanweisung enthalten.

Ein alternativer Kit für die vorstehend genannten Assayverfahren umfaßt die folgenden Komponenten:
a) Zellen, in denen zumindest unter bestimmten Bedingungen der sich an ein Ras-Protein anschließende Signalweg nicht aktiviert werden kann,
b) einen oder mehrere Transformations- oder Transfektionsvektoren, die mindestens eine DNA-Sequenz enthalten, die ein Fusionsprotein, wie vorstehend definiert, kodiert, wobei das Fusionsprotein eine dritte Domäne, die in der Lage ist, den inaktiven oder inaktivierbaren, sich an ein Ras-Protein anschließenden Signalweg in den Zellen zu aktivieren, umfaßt,
c) gegebenenfalls Reagenzien zur Transformation oder Transfektion der Zellen mit dem Transformations- oder Transfektionsvektor,
d) gegebenenfalls Reagenzien zur Detektion der phänotypischen Aktivierung des sich an ein Ras-Protein anschließenden Signaltransduktionsweges in diesen Zellen.

Ein weiterer alternativer Kit ermöglicht die Herstellung der Assayzelle mit einem Fusionsprotein, das eine individuell gewünschte zweite Domäne enthält. Er umfaßt folgende Komponenten:
a) Zellen, in denen zumindest unter bestimmten Bedingungen ein sich an ein Ras-Protein anschließender Signalweg nicht aktiviert werden kann,
b) einen Transformations- oder Transfektionsvektor, der in geeigneter Anordnung
   -- eine DNA-Sequenz, die eine erste Domäne eines Fusionsproteins, wie vorstehend definiert, kodiert,
   -- eine DNA-Sequenz, die eine dritte Domäne eines Fusionsproteins, wie vorstehend definiert und die in der Lage ist, den inaktiven oder inaktivierbaren, sich an ein Ras-Protein anschließenden Signalweg in den Zellen zu aktivieren, kodiert, und
   -- eine geeignet angeordnete Insertionsstelle zur funktionalen Insertion einer DNA-Sequenz, die eine zweite Domäne, wie vorstehend definiert, kodiert,
   aufweist, wobei nach Insertion einer DNA-Sequenz für die zweite Domäne der Vektor ein vollständiges Gen für ein Fusionsprotein, wie es vorstehend definiert wurde, umfaßt,
c) gegebenenfalls Reagenzien zur Transformation oder Transfektion der Zellen mit dem Transformations- oder Transfektionsvektor
d) gegebenenfalls Reagenzien zur Detektion der phänotypischen Aktivierung des sich an ein Ras-Protein anschließenden Signaltransduktionsweges in diesen Zellen.

Auch für die beiden letztgenannten Assays gilt, daß die in dem Kit enthaltenen Zellen bei einem vorgesehenen Nachweis einer Reportergenaktivität u.a. zusätzlich auch ein Konstrukt, umfassend eine Bindungsstelle für einen Transkriptionsfaktor, dessen Aktivierung infolge der Aktivierung des speziellen ras-Signalwegs, dessen Aktivierung durch den Assay nachgewiesen werden soll, erfolgt, einen Minimalpromotor und das Reportergen, wie vorstehend erläutert, enthalten können, oder es kann alternativ getrennt von den Zellen ein Transformations- oder Transfektionsvektor mit dem Transkriptionsfaktorbindungsstelle-Minimalpromotor-Reportergen-Konstrukt oder mit einem andersartigen Konstrukt, umfassend die Transkriptionsfaktorbindungsstelle und den Minimalpromotor und darüber hinaus eine geeignet angeordneten Insertionsstelle für ein frei wählbares Reportergen, vorgesehen werden.

Durch die Erfindung werden auch Kits für die erfindungsgemäßen Assayverfahren zum Nachweis, ob ein Polypeptid oder Protein eine Ligandenbindungsfunktion eines nukleären Rezeptors aufweist, bereitgestellt. Ein hierfür geeigneter Kit umfaßt erfindungsgemäße Zellen, wobei das darin enthaltene Fusionsprotein eine zweite Domäne, die ein Polypeptid oder Protein umfaßt, bei dem eine Ligandenbindungsfunktion eines nukleären Rezeptors vermutet wird, umfaßt.

Ein alternativer Kit umfaßt folgende Komponenten:
a) Zellen, in denen zumindest unter bestimmten Bedingungen ein sich an ein Ras-Protein anschließender Signalweg nicht aktiviert werden kann,
b) einen oder mehrere Transformations- oder Transfektionsvektoren, die mindestens eine DNA-Sequenz umfassen, die ein Fusionsprotein, wie vorstehend definiert, dessen zweite Domäne ein Polypeptid oder Protein, bei dem eine Ligandenbindungsfunktion eines nukleären Rezeptors vermutet wird, umfaßt und dessen dritte Domäne in der Lage ist, den inaktiven oder inaktivierbaren, sich an ein Ras-Protein anschließenden Signalweg in den Zellen zu aktivieren, kodiert,
c) gegebenenfalls Reagenzien zur Transformation oder Transfektion der Zellen mit dem Transformations- oder Transfektionsvektor,
d) gegebenenfalls Reagenzien zur Detektion der phänotypischen Aktivierung des sich an ein Ras-Protein anschließenden Signaltransduktionsweges in diesen Zellen.

Ein alternativer Kit zur Verwendung in dem genannten Assay ermöglicht die gezielte Bereitstellung einer Assayzelle mit einem Fusionsprotein, das ein gewünschtes, auf seine Ligandenbindungsfunktion zu untersuchendes Polypeptid oder Protein als zweite Domäne umfaßt. Ein solcher Kit umfaßt:
a) Zellen, in denen zumindest unter bestimmten Bedingungen ein sich an ein Ras-Protein anschließender Signalweg nicht aktiviert werden kann,
b) einen Transformations- oder Transfektionsvektor, der in geeigneter Anordnung
   -- eine DNA-Sequenz, die eine erste Domäne eines Fusionsproteins, wie vorstehend definiert, kodiert, und
   -- eine DNA-Sequenz, die eine dritte Domäne eines Fusionsproteins, wie vorstehend definiert und die in der Lage ist, den inaktiven oder inaktivierbaren, sich an ein Ras-Protein anschließenden Signalweg in den Zellen zu aktivieren, kodiert, und
   -- eine geeignet angeordnete Insertionsstelle zur funktionalen Insertion einer DNA-Sequenz, die eine zweite Domäne, enthaltend ein Polypeptid oder Protein, bei dem eine Ligandenbindungsfunktion eines nukleären Rezeptors vermutet wird, kodiert,
   aufweist, wobei nach Insertion einer DNA-Sequenz für die zweite Domäne der Vektor ein vollständiges Gen für ein Fusionsprotein, wie vorstehend definiert, bei dem die zweite Domäne ein Polypeptid oder Protein enthält, bei dem eine Ligandenbindungsfunktion eines nukleären Rezeptors vermutet wird, umfaßt,
c) gegebenenfalls Reagenzien zur Transformation oder Transfektion der Zellen mit dem Transformations- oder Transfektionsvektor,
d) gegebenenfalls Reagenzien zur Detektion der phänotypischen Aktivierung des sich an ein Ras-Protein anschließenden Signaltransduktionsweges in diesen Zellen.

Bei einem vorgesehenen Nachweis einer Reportergenaktivität enthalten in einer Ausführungsform die Zellen in den vorstehend genannten Kits zusätzlich ein Konstrukt, umfassend eine Bindungsstelle für einen Transkriptionsfaktor, dessen Aktivierung infolge einer Aktivierung des speziellen ras-Signalwegs, dessen Aktivierung durch den Assay nachgewiesen werden soll, erfolgt, einen Minimalpromotor und das Reportergen, wie vorstehend erläutert, oder es kann alternativ getrennt von den Zellen ein Transformations- oder Transfektionsvektor mit dem Transkriptionsfaktorbindungsstelle-Minimalpromotor-Reportergen-Konstrukt oder mit einem andersartigen Konstrukt, umfassend die Transkriptionsfaktorbindungsstelle und den Minimalpromotor und darüber hinaus eine geeignet angeordneten Insertionsstelle für ein frei wählbares Reportergen, vorgesehen werden.

In einer bevorzugten Ausführungsform der Erfindung umfassen die erfindungsgemäßen Kits die Zellen immobilisiert auf einem festen Träger, wie vorstehend erläutert, insbesondere auf Biochips. Für Massenscreens ist insbesondere die Immobilisierung der Zellen in den individuellen Vertiefungen von Mikrotiterplatten geeignet, so daß auf einer solchen Platte eine Vielzahl von separaten Assayverfahren durchgeführt werden können. Es ist hierbei auch möglich, unterschiedliche erfindungsgemäße Zellen, d.h. insbesondere Zellen mit unterschiedlicher zweiter Domäne, in Vertiefungen in jeweils bestimmten Abschnitten auf ein und derselben Mikrotiterplatte vorzusehen.

Befinden sich die Zellen in dem Kit immobilisiert an einem festen Träger, so kann es insbesondere bei Nachweis einer Reportergenaktivität oder eines Reporterproteins erforderlich oder hilfreich sein, die Zellen vor der Nachweisreaktion zu solubilisieren, d.h. sie von dem Träger abzulösen und ggf. auch aufzubrechen. Für diesen Fall können die unter d) genannten Reagenzien zur Detektion der phänotypischen Aktivierung des ras-Signaltransduktionsweges auch geeignete Solubilisierungsreagenzien, die insbesondere ein oder mehrere grenzflächenaktive Mittel oder Tenside enthalten, umfassen.

Außerdem erstreckt sich die Erfindung auch auf
- Liganden für einen Bindungsabschnitt eines Rezeptors,
- Verbindungen, die in der Lage sind, eine Bindungsaktivität eines Ligandenbindungsabschnitts eines Rezeptors gegenüber einem Liganden zu verändern, (im Folgenden als "Modifizierungsverbindungen" bezeichnet) sowie
- Polypeptide oder Proteine, die eine Ligandenbindungsfunktion eines Rezeptors aufweisen,
die mittels eines der Assayverfahren der Erfindung bestimmt oder ermittelt worden sind, sowie Zusammensetzungen, die diese Liganden, Verbindungen und/oder Polypeptide oder Proteine enthalten.

Hinsichtlich Polypeptiden oder Proteinen, die eine Ligandenbindungsfunktion eines Rezeptors aufweisen und zur Erzeugung des Fusionsproteins, wie in Anspruch 1 definiert, von einem natürlich aufgefundenen oder synthetisch erzeugten Molekül abgeleitet worden sind, umfaßt die Erfindung sowohl das in den erfindungsgemäß eingesetzten Fusionsproteinen enthaltene Fragment mit Ligandenbindungsfunktion als auch das Ausgangsfragment- bzw. - molekül. Nur der Klarheit halber sei diesbezüglich angemerkt, daß die Erzeugung des Fusionsproteins in der Regel durch Expression einer dieses Fusionsprotein kodierenden Nukleinsäuresequenz in einer Zelle erfolgt. Das Ableiten eines Polypeptids oder Proteins mit Ligandenbindungsfunktion eines Rezeptors von einem größeren Ausgangsmolekül erfolgt dementsprechend in der Regel in analoger Weise auf Nukleinsäure-Ebene, indem lediglich ein oder mehrere Abschnitte der das Ausgangsmolekül kodierenden Nukleinsäuresequenz, gegebenenfalls unter darauffolgender Klonierung zur Anfügung von Abschnitten, die weitere Fusionsproteinkomponenten oder -abschnitte kodieren, für die Expression des Fusionsproteins genutzt werden. Im Rahmen des Ableitens können auch eine oder mehrere geringfügige Nukleinsäuresequenzmodifizierungen in der Ausgangssequenz oder dem oder den Nukleinsäureabschnitt(en) vorgenommen werden, bevorzugt solcher Art, daß das resultierende Nukleinsäuremolekül unter stringenten Bedingungen noch mit dem jeweiligen Ausgangsnukleinsäuremolekül hybridisiert.

Die Erfindung umfaßt dementsprechend auch ein Verfahren zur Identifizierung von Polypeptiden oder Proteinen, insbesondere Rezeptoren, die eine Ligandenbindungsfunktion eines Rezeptors aufweisen, welches umfaßt:
- eine erfindungsgemäße Zelle mit einem Fusionsprotein, das die in Anspruch 1 beschriebenen Merkmale aufweist und die Gesamtheit eines derartigen Polypeptids oder Proteins oder einen Teil eines derartigen Polypeptids oder Proteins, der mutmaßlich die für die Ligandenbindungsfunktion essentiellen Sequenzabschnitte enthält, umfaßt, herzustellen und
- mittels dieser Zelle das erfindungsgemäße in vivo-Assayverfahren zum Nachweis, ob ein Polypeptid oder Protein eine Ligandenbindungsfunktion eines nukleären Rezeptors aufweist, auszuführen.
sowie die das durch dieses Verfahren identifizierten Moleküle.
Die Erfindung erstreckt sich gleichfalls auf
- die Verwendung der vorstehend genannten, durch die erfindungsgemäßen Assay-Verfahren identifizierten Liganden, Modifizierungsverbindungen und Polypeptide bzw. Proteine als Arzneimittel, gegebenenfalls nach Formulierung mit in diesem Bereich üblichen Hilfs- und/oder Trägerstoffen, sowie
- die Verwendung der Liganden, Modifizierungsverbindungen und Polypeptide bzw. Proteine als Leitsubstanzen zur Entwicklung von davon - insbesondere durch Derivatisierung - abgeleiteten Liganden, Modifizierungsverbindungen und Polypeptiden bzw. Proteinen, insbesondere solchen mit entsprechender oder verbesserter Aktivität gegenüber der jeweiligen Leitsubstanz.

Somit umfaßt die Erfindung auch ein Verfahren zur Herstellung von Liganden, Modifizierungsverbindungen, Polypeptiden oder Proteinen durch ein- oder mehrfache Derivatisierung ausgehend von mittels der erfindungsgemäßen Assayverfahren identifizierten Liganden, Modifizierungsverbindungen, Polypeptiden oder Proteinen. Dieses Verfahren kann gegebenenfalls zusätzlich die Schritte umfassen,
- auch die durch die Derivatisierung erhaltenen Liganden, Modifizierüngsverbindungen, Polypeptide oder Proteine mittels der erfindungsgemäßen Assay-Verfahren auf Ligandenfunktion bzw. Ligandenbindungsfunktion zu testen, und/oder
- die durch die Derivatisierung erhaltenen Liganden, Modifizierungsverbindungen, Polypeptide oder Proteine in üblicher

Weise als Arzneimittel zu formulieren.
Des weiteren erstreckt sich die Erfindung auch auf die durch dieses Verfahren erhaltenen Liganden, Modifizierungsverbindungen, Polypeptide und Proteine, d.h. die durch dieses Verfahren erhaltenen funktionellen Derivate.

Für eine Verwendung als Gentherapeutika, die die Expression eines Polypeptids oder Proteins, das eine Ligandenbindungsfunktion eines Rezeptors, insbesondere nukleären Rezeptors aufweist, insbesondere in menschlichen oder tierischen Zellen bewirken sollen, umfaßt die Erfindung des weiteren Nukleinsäuremoleküle, die ausgehend von einem mittels der erfindungsgemäßen Assay-, Identifizierungs-, Screening- oder Herstellungsverfahren identifizierten Polypeptid oder Protein, insbesondere Rezeptor, durch ein Verfahren erhalten werden, das die Bereitstellung des das Polypeptid oder Protein kodierenden Gens oder eines Teils davon, der zumindest die für die Aktivität des kodierten Polypeptids oder Proteins essentiellen Nukleinsäuresequenzabschnitte umfaßt, in im wesentlichen reiner Form, d.h. insbesondere im wesentlichen frei von anderen Nukleinsäuren, die für die Verwendung als Gentherapeutikum nicht erforderlich oder gar abträglich sind, umfaßt. Dieses Verfahren kann, sofern noch nicht bekannt, die vorab erfolgende Identifizierung des Gens, das dieses Polypeptid oder Protein kodiert, erfordern. Insbesondere kann das Verfahren zusätzlich auch die folgenden Schritte umfassen:
- sofern noch nicht bekannt, die Aminosäuresequenz des Polypeptids oder Proteins, insbesondere Rezeptors, zu bestimmen, und/oder
- sofern noch nicht bekannt, das Gen, das dieses Polypeptid oder Protein kodiert, zu identifizieren und zumindest die Sequenz der kodierenden Abschnitte dieses Gens zu bestimmen,
- gegebenenfalls Modifizierungen in der erhaltenen Nukleinsäuresequenz vorzunehmen, beispielsweise zum Anpassen der Codonnutzung an diejenige eines gewünschten Empfängerorganismus, zum Einführen von Mutationen oder zur Entfernung von Intron-Sequenzen, und
- die gegebenenfalls modifizierte Nukleinsäuresequenz in Form eines Gentherapeutikums zu formulieren.

In einem gegenwärtig bevorzugt verwendeten experimentellen System der Erfindung, fehlt einem mutierten Ras-Protein (Ha-Ras (61L), welches ein Bestandteil des von der Nukleinsäuresequenz kodierten Fusionsproteins ist, die Farnesylierungssequenz, welche für eine Membranlokalisierung des Proteins sorgt. Des weiteren wird als Zellsystem, welches in einem ras- oder ras-ähnlichen Signaltransduktionsweg inaktiv ist, der Hefestamm cdc25-2 verwendet. Wie erläutert, ist in diesen Zellen das Ras-Protein bei einer restriktiven Temperatur von 33-37°C, typischerweise 36°C, infolge der Abwesenheit eines funktionellen Guaninnukleotid-Austauschfaktors (GEF; "guanyl nucleotide exchange factor") nicht funktionell. Die Expression eines funktionellen, membranständigen Ras-Proteins in Fusion mit einem nukleären Rezeptor und/oder Teilen von solchen kann dadurch detektiert werden, daß die Hefezellen unabhängig von der Anwesenheit eines funktionellen GEF-Proteins bei den genannten restriktiven Temperaturen wachsen können, sofern ein passender Ligand des exprimierten nukleären Rezeptors vorhanden ist.
Um die Erfindung weiter zu erläutern, folgt nun die Beschreibung eines exemplarischen Beispiels.

### Material und Methoden

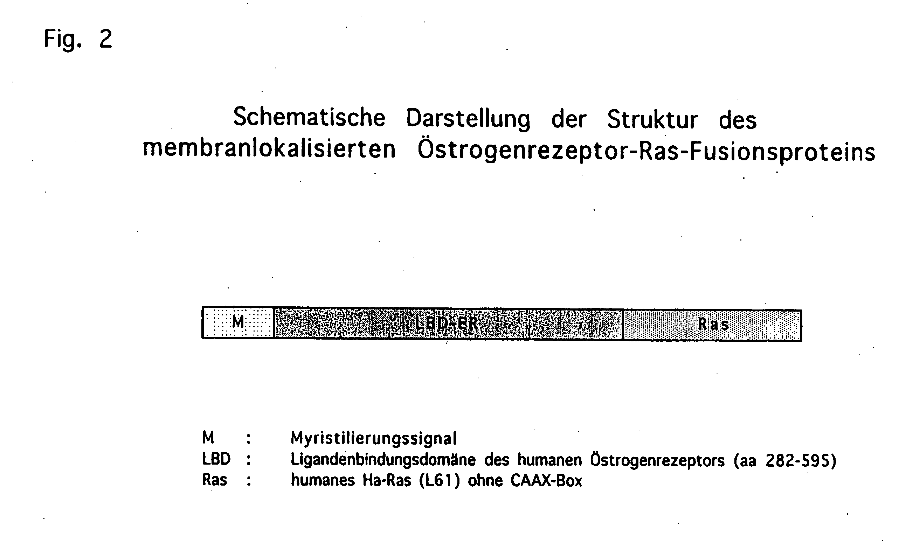

Als Basisvektor dient ein Vektor mit einem Markergen (Ura) und einem Galaktose-induzierbaren Promotor (GALI) für das zu exprimierende Fusionsgen. Wie in Fig. 2 schematisch gezeigt, kodiert die zu exprimierende DNA-Sequenz:
1. ein Myristylierungssignal,
2. die Ligandenbindungsdomäne des humanen Östrogenrezeptors (Aminosäuren 282-595),
3. das humane Ha-Ras (L61), welches konstitutiv aktiv ist und dem die sogenannte CAAX-Box, das Farnesylierungssignal zur Membranlokalisierung fehlt.

### Hefewachstum und -manipulation

Es wurden konventionelle Hefe-Transformations- und - manipulationsprotokolle (siehe z.B. Hill et al. (1991), NAR 19, 5791) verwendet. Die Zellen wurden entweder auf einem Glucoseminimalmedium, welches die erforderlichen Aminosäuren und Nukleotide (20 mg/l Histidin, 100 mg/l Leucin, 20 mg/l Tryptophan, 20 mg/l Uracil, 10 mg/l Adeninsulfat), 2% Glucose, 0,5% NH₄SO₄ ,0,17% Hefeextrakt und 4% Agar enthält, oder auf Galaktosemedium (1,7 g/l Yeast Nitrogen ohne Aminosäuren, 5 g/l Ammoniumsulfat, 30 g/l Galaktose (> 99%) 20 g/l D-Raffinose, 20 g/l Glycerin (100%), 30 g/l Bacto-Agar) ausplattiert.

Zur Kontrolle wurden Klone auf YPD-Medium (1% Hefeextrakt, 2% Bactotrypton und 2% Glucose) ausplattiert. YPD-Medium enthält keine Galactose, so daß keine Expression des Fusionsproteins erfolgen sollte. Erfolgreich transformierte Klone zeigten bei Züchtung auf diesem Medium bei Zusatz des Liganden Östrogen, wie erwartet, keine Zellvermehrung.

Replika-Plattierungen wurden mit einem Samt-Replikaplattierer durchgeführt. Die Zellen wurden nach der Transformation mit dem vorstehend beschriebenen Nukleinsäurevektor auf Glucoseplatten ausplattiert und bei einer nicht-restriktiven Temperatur von 25°C drei bis vier Tage inkubiert. Anschließend wurden verschiedene Flüssigmedien (mit und ohne Östrogen) mit jeweils 3 unabhängigen Klonen angeimpft und bei 37°C 12 bis 36 h inkubiert und anschließend das Wachstum der Hefen in den verschiedenen Flüssigmedien durch photometrische Messung der optischen Dichte bei 600 nm detektiert. Die folgende Tabelle gibt einen Überblick über die gewählten Medien und die erzielten Resultate:

**TABELLE 1**

| Detektion des Wachstums von cdc25-2-Hefezellen in Abhängigkeit von verschiedenen Östrogenkonzentrationen im Medium | | | | | | | | |
|---|---|---|---|---|---|---|---|---|
| | Glu-Medium | Glu-Medium | Gal-Medium | Gal-Medium | Gal-Medium | Gal-Medium | Gal-Medium | Gal-Medium |
| | -E | + E | - E | + E | + E | + E | + E | + E |
| | | (10⁻⁵M) | | (10⁻⁶M) | (10⁻⁷M) | (10⁻⁸M) | (10⁻⁹M) | (10⁻¹⁰M) |
| 25°C | + | + | + | + | + | + | + | + |
| 37°C | - | - | - | + | + | + | +/- | - |

| | | | | | | | | |
|---|---|---|---|---|---|---|---|---|
| Glu-Medium: Glucosemedium Gal-Medium: Galactosemedium E: östrogen -: kein Wachstum der Hefen +: Wachstum der Hefen +/-: schwaches Wachstum der Hefen | | | | | | | | |

### Literatur:

Bargmann, Cell, 90: 585-587, 1997;
Current Protocols in Molecular Biology, 1991;
Evans, Science, 240: 889-895, 1988;
Hill, NAR, 19: 5791, 1991;
Kastner et al., Cell, 83: 859-869, 1995;
Pratt, Endocr. Rev., 18: 306-360, 1997;
Sambrook, J., Fritsch, E.F., Maniatis, T. (1989), Molecular Cloning. A Laboratory Handbook, Cold Spring Harbor Laboratory, New York;
Schlessinger, TIBS, 18: 273-275, 1993;
Seed, Nature Medicine, 4: 1004-1005, 1998.

## Patentansprüche

1. Fusionsprotein, umfassend mindestens drei Domänen, wobei
- eine erste Domäne, welche eine Membranlokalisierung des Fusionsproteins in einem zellulären Kontext vermittelt und eine Aminosäuresequenz eines Membranlokalisierungssignals oder einer Transmembrandomäne aufweist oder davon abgeleitet ist,
- eine zweite Domäne, welche eine Ligandenbindungsfunktion eines nukleären Rezeptors aufweist oder mutmaßlich aufweist und eine Aminosäuresequenz des Rezeptorabschnitts eines Steroid-Rezeptors, eines Orphan-Rezeptors, eines Vitamin-Rezeptors, eines Thyroxin-Rezeptors, eines Dioxin-Rezeptors oder eines Retinsäure-Rezeptors umfasst oder davon abgeleitet ist,
- eine dritte Domäne, welche eine Aktivität aufweist, die in der Lage ist, eine sich an ein Ras-Protein anschließenden Signalweg in einer Zelle zu aktivieren und eine Aminosäuresequenz umfasst, die von der Aminosäuresequenz eines in der Natur vorkommenden Ras-Proteins oder eines in der Natur vorkommenden Guaninnukleotid-Austauschfaktors abgeleitet ist,
**dadurch gekennzeichnet,**
**dass** bei fehlender Bindung von Ligand an die zweite Domäne des Fusionsproteins die dritte Domäne ihre Aktivität zur Aktivierung eines sich an ein Ras-Protein anschließenden Signalwegs in der Zelle trotz Membranlokalisierung nicht ausüben kann, jedoch bei Bindung von Ligand an die zweite Domäne eine Konformationsänderung mit Auswirkungen auf die dritte Domäne bewirkt wird, so dass die dritte Domäne ihre Aktivität zur Aktivierung eines sich an ein Ras-Protein anschließenden Signalwegs in der Zelle ausüben kann.

2. Fusionsprotein nach Anspruch 1,
**dadurch gekennzeichnet,**
**dass** die erste Domäne die Aminosäuresequenz eines Farnesylierungssignals, Myristylierungssignals oder Prenylierungssignals aufweist oder davon abgeleitet ist.

3. DNA-Molekül, das das Fusionsprotein nach Anspruch 1 oder 2 kodiert.

4. Vektor, insbesondere Plasmid, Cosmid, Viren- oder Phagengenom, umfassend mindestens ein DNA-Molekül nach Anspruch 3.

5. Einzellige eukaryontische Zelle, umfassend ein Fusionsprotein nach Anspruch 1 oder 2,
**dadurch gekennzeichnet,**
**dass** bei Bindung von Ligand an die zweite Domäne des Fusionsproteins die dritte Domäne ihre Aktivität zur Aktivierung eines sich an ein Ras-Protein anschließenden Signalwegs in der Zelle trotz Membranlokalisierung nicht ausüben kann, jedoch bei Abdissoziierung des Liganden von der zweiten Domäne eine Konformationsänderung mit Auswirkungen auf die dritte Domäne bewirkt wird, so dass die dritte Domäne ihre Aktivität zur Aktivierung eines sich an ein Ras-Protein anschließenden Signalwegs in der Zelle ausüben kann und dass in Abwesenheit von Fusionsprotein zumindest unter bestimmten Bedingungen ein sich an ein Ras-Protein anschließender Signalweg in der Zelle nicht aktiviert werden kann.

6. Zelle nach Anspruch 5,
**dadurch gekennzeichnet,**
**dass** die Zelle eine Hefezelle ist.

7. Zelle nach Anspruch 6,
**dadurch gekennzeichnet,**
**dass** die Zelle eine zellwandlose Hefezelle ist.

8. Verwendung einer Zelle nach Anspruch 5 in einem *in vivo*-Assay zum Nachweis, ob eine Verbindung in der Lage ist, eine Bindungsaktivität eines Rezeptorabschnitts eines nuklearen Rezeptors gegenüber einem Liganden zu verändern, **gekennzeichnet durch** die folgenden Schritte:
(a) Inkontaktbringen des Liganden in Anwesenheit der Verbindung mit Zellen nach Anspruch 5 unter Bedingungen, bei denen die Verbindung in die Zellen diffundieren kann oder sie von den Zellen produziert wird und bei denen in Abwesenheit von Fusionsprotein ein sich an ein Ras-Protein anschließender Signalweg in den Zellen nicht aktiviert werden kann,
(b) Untersuchen, ob und gegebenenfalls in welchem Ausmaß eine Aktivierung des sich an ein Ras-Protein anschließenden Signalweges erfolgt ist,
(c) Vergleichen des Untersuchungsergebnisses von Schritt (b) mit einem Untersuchungsergebnis, das bei Ausführen des Assay in Abwesenheit der Verbindung erhalten wird.

9. Verwendung nach Anspruch 8, wobei in Schritt (a) Zellen, bei denen der inaktive, sich an ein Ras-Protein anschließende Signalwegs ein Signalweg ist, der auf den Zellzyklus einwirkt und dessen Aktivierung für die Zellvermehrung essenziell ist, eingesetzt werden und Schritt (b) umfasst, zu untersuchen, ob und gegebenenfalls in welchem Ausmaß die Zellen unter den genannten Bedingungen vermehrungsfähig sind, wobei in dem Falle, wo der Vergleich in Schritt (c) ergibt, dass in Anwesenheit der Verbindung eine stärkere Zellvermehrung auftritt, eine Agonistenwirkung der Verbindung angezeigt wird
und in dem Falle, wo der Vergleich in Schritt (c) ergibt, dass in Anwesenheit der Verbindung eine geringere Zellvermehrung auftritt, eine Antagonistenwirkung der Verbindung angezeigt wird.

10. Verwendung einer Zelle nach Anspruch 5 in einem *in vivo-*Assay zum Nachweis, ob ein Polypeptid oder Protein eine Ligandenbindungsfunktion eines nukleären Rezeptors aufweist, **gekennzeichnet durch** die folgenden Schritte:
(a) Inkontaktbringen von Zellen nach Anspruch 5 mit dem Liganden unter Bedingungen, bei denen in Abwesenheit des Fusionsproteins ein sich an ein Ras-Protein anschließender Signalweg in den Zellen nicht aktiviert werden kann, wobei das in den Zellen enthaltene Fusionsprotein eine zweite Domäne, die das zu untersuchende Polypeptid oder Protein umfasst, und eine dritte Domäne, die bei Bindung von Ligand die zweiten Domäne in der Lage ist, den inaktiven, sich an ein Ras-Protein anschließenden Signalweg in den Zellen zu aktivieren, umfasst,
(b) Untersuchen, ob eine Aktivierung des sich an ein Ras-Protein anschließenden Signalwegs erfolgt ist,
wobei ein Nachweis der Aktivierung des sich an ein Ras-Protein anschließenden Signalwegs anzeigt, dass die zweite Domäne des Fusionsproteins und dementsprechend das zu untersuchende Polypeptid oder Protein eine Ligandenbindungsfunktion eines nukleären Rezeptors aufweist.

11. Kit zur Verwendung in einem Assay oder Screeningverfahren nach einem der Ansprüche 8 bis 10,
**dadurch gekennzeichnet,**
**dass** er
(a) Zellen nach Anspruch 5 umfasst sowie
(b) gegebenenfalls einen oder mehrere Transformations- oder Transfektionsvektoren, die mindestens eine DNA-Sequenz enthalten, die ein Fusionsprotein nach Anspruch 1 oder 2 kodiert,
(c) gegebenenfalls Reagenzien zur Transformation oder Transfektion der Zellen mit dem Transformations- oder Transfektionsvektor,
(d) gegebenenfalls Reagenzien zur Detektion der phänotypischen Aktivierung des sich an ein Ras-Protein anschließenden Signaltransduktionsweges in diesen Zellen.

12. Kit zur Verwendung in einem Assay nach einem der Ansprüche 8 bis 10,
**dadurch gekennzeichnet,**
**dass** er folgende Bestandteile umfasst:
(a) Zellen nach Anspruch 5,
(b) einen Transformations- oder Transfektionsvektor, der in geeigneter Anordnung
- eine DNA-Sequenz, die eine erste Domäne eines Fusionsproteins, wie in Anspruch 1 oder 2 definiert, kodiert,
- eine DNA-Sequenz, die eine dritte Domäne eines Fusionsproteins, wie in Anspruch 1 oder 2 definiert, kodiert, und
- eine geeignet angeordnete Insertionsstelle zur funktionalen Insertion einer DNA-Sequenz, die eine zweite Domäne, wie in Anspruch 1 oder 2 definiert, kodiert,
aufweist, wobei nach Insertion einer DNA-Sequenz für die zweite Domäne der Vektor ein vollständiges Gen für ein Fusionsprotein nach Anspruch 1 umfasst,
(c) gegebenenfalls Reagenzien zur Transformation oder Transfektion der Zellen mit dem Transformations- oder Transfektionsvektor,
(d) gegebenenfalls Reagenzien zur Detektion der phänotypischen Aktivierung des sich an ein Ras-Protein anschließenden Signaltransduktionsweges in diesen Zellen.

13. Kit zur Verwendung in einem Assay nach Anspruch 10,
**dadurch gekennzeichnet,**
**dass** er folgende Bestandteile umfasst:
(a) Zellen nach Anspruch 5,
(b) einen Transformations- oder Transfektionsvektor, der in geeigneter Anordnung
- eine DNA-Sequenz, die eine erste Domäne eines Fusionsproteins, wie in Anspruch 1 oder 2 definiert, kodiert, und
- eine DNA-Sequenz, die eine dritte Domäne eines Fusionsproteins, wie in Anspruch 1 oder 2 definiert, kodiert, und
- eine geeignet angeordnete Insertionsstelle zur funktionalen Insertion einer DNA-Sequenz, die eine zweite Domäne des Fusionsproteins nach Anspruch 1 oder 2 kodiert,
aufweist, wobei nach Insertion einer DNA-Sequenz für die zweite Domäne der Vektor ein vollständiges Gen für ein Fusionsprotein nach Anspruch 1 oder 2 umfasst, bei dem eine Ligandenbindungsfunktion eines nukleären Rezeptors vermutet wird,
(c) gegebenenfalls Reagenzien zur Transformation oder Transfektion der Zellen mit dem Transformations- oder Transfektionsvektor,
(d) gegebenenfalls Reagenzien zur Detektion der phänotypischen Aktivierung des sich an ein Ras-Protein anschließenden Signaltransduktionsweges in diesen Zellen.

14. Kit nach Anspruch 13,
zusätzlich umfassend:
einen Transformations- oder Transfektionsvektor mit einem Konstrukt, umfassend
- eine Bindungsstelle für einen Transkriptionsfaktor, dessen Aktivierung infolge einer Aktivierung eines speziellen Ras-Signalwegs, dessen Aktivierung durch den Assay nachgewiesen werden soll, erfolgt,
- einen Minimalpromotor und
- eine für eine durch den Minimalpromotor gesteuerte Expression geeignet angeordnete Insertionsstelle für eine Insertion eines Gens für ein Reporterprotein,
- wobei der Minimalpromotor infolge einer Bindung des aktivierten Transkriptionsfaktors an seine Bindungsstelle aktiviert wird.

## Claims

1. Fusion protein comprising at least three domains, where
- a first domain mediates a membrane localization of the fusion protein in a cellular context and has an amino acid sequence of a membrane localization signal or of a transmembrane domain or is derived therefrom,
- a second domain has, or presumably has, a ligand binding function of a nuclear receptor and comprises an amino acid sequence of the receptor section of a steroid receptor, an orphan receptor, a vitamin receptor, a thyroxine receptor, a dioxin receptor or a retinoic acid receptor or is derived therefrom,
- a third domain has an activity which is able to activate a signal pathway connected to a Ras protein in a cell and comprises an amino acid sequence which is derived from the amino acid sequence of a naturally occurring Ras protein or of a naturally occurring guanine nucleotide exchange factor,
**characterized in that**
when there is a lack of binding of ligand to the second domain of the fusion protein, the third domain cannot exert its activity to activate a signal pathway connected to a Ras protein in the cell despite membrane localization, but when ligand binds to the second domain it causes a change in conformation which effects the third domain such that the third domain can exert its activity to activate a signal pathway connected to a Ras protein in the cell.

2. Fusion protein according to claim 1
**characterized in that**
the first domain has the amino sequence of a farnesylation signal, myristylation signal or prenylation signal or is derived therefrom.

3. DNA molecule which encodes the fusion protein according to claim 1 or 2.

4. Vector, in particular plasmid, cosmid, viral or phage genome comprising at least one DNA molecule according to claim 3.

5. Single-celled eukaryotic cell comprising a fusion protein according to claim 1 or 2,
**characterized in that**
when a ligand binds to the second domain of the fusion protein, the third domain cannot exert its activity to activate a signal pathway connected to a Ras protein in the cell despite membrane localization, but when the ligand dissociates from the second domain it causes a change in conformation which has effects on the third domain such that the third domain can exert its activity to activate a signal pathway connected to a Ras protein in the cell and that in the absence of the fusion protein a signal pathway connected to a Ras protein cannot be activated in the cell at least under certain conditions.

6. Cell according to claim 5,
**characterized in that**
the cell is a yeast cell.

7. Cell according to claim 6,
**characterized in that**
the cell is a yeast cell without a cell wall.

8. Use of a cell according to claim 5 in an *in vivo* assay for determining whether a compound is able to change a binding activity of a receptor section of a nuclear receptor with respect to a ligand, **characterized by** the following steps:
(a) contacting the ligand in the presence of the compound with cells according to claim 5 under conditions which allow the compound to diffuse into the cells or to be produced by the cells and under which a signal pathway connected to a Ras protein in the cells cannot be activated in the absence of the fusion protein,
(b) examining whether and optionally to what extent an activation of the signal pathway connected to a Ras protein has taken place,
(c) comparing the test result of step (b) with a test result which has been obtained when the assay is carried out in the absence of the compound.

9. Use according to claim 8, where in step (a) cells are used in which the inactive signal pathway connected to a Ras protein is a signal pathway which has an effect on the cell cycle and whose activation is essential for cell proliferation and step (b) comprises examining whether and optionally to what extent the cells can proliferate under the said conditions and if the result of the comparison in step (c) is that a stronger cell proliferation takes place in the presence of the compound, this indicates an agonist effect of the compound and if the result of the comparison in step (c) is that a decreased cell proliferation occurs in the presence of the compound, this indicates an antagonist effect of the compound.

10. Use of a cell according to claim 5 in an *in vivo* assay to determine whether a polypeptide or protein has a ligand binding function of a nuclear receptor,
**characterized by** the following steps:
(a) contacting cells according to claim 5 with the ligand under conditions under which a signal pathway connected to a Ras protein in the cells cannot be activated in the absence of the fusion protein where the fusion protein present in the cells contains a second domain which comprises the polypeptide or protein to be examined, and a third domain which is able to activate the inactive signal pathway connected to a Ras protein in the cells when the ligand binds,
(b) examining whether an activation of the signal pathway connected to a Ras protein has taken place,
wherein a detection of the activation of the signal pathway connected to a Ras protein indicates that the second domain of the fusion protein and accordingly the polypeptide or protein to be examined has a ligand binding function of a nuclear receptor.

11. Kit for use in an assay or screening method according to one of the claims 8 to 10,
**characterized in that** it comprises
(a) cells according to claim 5 and
(b) optionally one or more transformation or transfection vectors which contain at least one DNA sequence which encodes a fusion protein according to claim 1 or 2,
(c) optionally reagents for transforming or transfecting the cells with the transformation or transfection vector,
(d) optionally reagents for detecting the phenotypical activation of the signal transduction pathway connected to a Ras protein in these cells.

12. Kit for use in an assay according to one of the claims 8 to 10,
**characterized in that**
it comprises the following components
(a) cells according to claim 5,
(b) a transformation or transfection vector which has in a suitable arrangement:
- a DNA sequence which encodes a first domain of a fusion protein as defined in claim 1 or 2,
- a DNA sequence which encodes a third domain of a fusion protein as defined in claim 1 or 2 and
- a suitably arranged insertion site for the functional insertion of a DNA sequence which encodes a second domain as defined in claim 1 or 2,
wherein, after insertion of a DNA sequence for the second domain, the vector comprises a complete gene for a fusion protein according to claim 1,
(c) optionally reagents for transforming or transfecting the cells with the transformation or transfection vector,
(d) optionally reagents for detecting the phenotypical activation of the signal transduction pathway connected to a Ras protein in these cells.

13. Kit for use in an assay according to claim 10,
**characterized in that**
it comprises the following components
(a) cells according to claim 5,
(b) a transformation or transfection vector which has in a suitable arrangement
- a DNA sequence which encodes a first domain of a fusion protein as defined in claim 1 or 2, and
- a DNA sequence which encodes a third domain of a fusion protein as defined in claim 1 or 2 and
- a suitably arranged insertion site for the functional insertion of a DNA sequence which encodes a second domain of the fusion protein according to claim 1 or 2,
wherein, after insertion of a DNA sequence for the second domain, the vector comprises a complete gene for a fusion protein according to claim 1 or 2 which is suspected to have a ligand binding function of a nuclear receptor,
(c) optionally reagents for transforming or transfecting the cells with the transformation or transfection vector
(d) optionally reagents for detecting the phenotypical activation of the signal transduction pathway connected to a Ras protein in these cells.

14. Kit according to claim 13,
additionally comprising:
a transformation or transfection vector containing a construct comprising
- a binding site for a transcription factor whose activation results from an activation of a specific Ras signal pathway whose activation is to be detected by the assay,
- a minimal promoter and
- an insertion site, suitably arranged for a controlled expression by the minimal promoter, for insertion of a gene for a reporter protein,
- wherein the minimal promoter is activated as a result of a binding of the activated transcription factor to its binding site.

## Revendications

1. Protéine de fusion, comprenant au moins trois domaines, où
- un premier domaine, qui fournit une localisation membranaire de la protéine de fusion dans un contexte cellulaire et qui présente une séquence des acides aminés d'un signal de localisation membranaire ou d'un domaine transmembranaire, ou qui dérive de celle-ci,
- un deuxième domaine, qui présente ou présente probablement une fonction de liaison d'un ligand d'un récepteur nucléaire et comprend une séquence des acides aminés du segment récepteur d'un récepteur de stéroïde, d'un récepteur d'orphane, d'un récepteur de vitamine, d'un récepteur de thyroxine, d'un récepteur de dioxine ou d'un récepteur de d'acide rétinoïque, ou qui dérive de celle-ci,
- un troisième domaine, qui présente une activité, qui est en mesure d'activer une voie de signal se raccordant à une protéine Ras dans une cellule et qui comprend une séquence des acides aminés, qui dérive de la séquence des acides aminés d'une protéine Ras naturelle ou d'un facteur d'échange de nucléotide guanine naturel,
**caractérisée en ce**
**que**, en l'absence d'une liaison de ligand sur le deuxième domaine de la protéine de fusion, le troisième domaine ne peut pas exercer son activité d'activation d'une voie de signal se raccordant à une protéine Ras dans la cellule malgré la localisation membranaire, mais lors de la liaison d'un ligand sur le deuxième domaine, une modification de conformation s'effectue avec actions sur le troisième domaine, de sorte que le troisième domaine peut exercer son activité d'activation d'une voie de signal se raccordant à une protéine Ras dans la cellule.

2. Protéine de fusion selon la revendication 1,
**caractérisée en ce**
**que** le premier domaine présente la séquence des acides aminés d'un signal de farnésylation, d'un signal de myristylation ou d'un signal de prénylation ou dérive de celle-ci.

3. Molécule d'ADN, qui code la protéine de fusion selon la revendication 1 ou 2.

4. Vecteur, en particulier plasmide, cosmide, génome de virus ou de phage, comprenant au moins une molécule d'ADN selon la revendication 3.

5. Cellule eucaryote individuelle, comprenant une protéine de fusion selon la revendication 1 ou 2,
**caractérisée en ce**
**que** lors de la liaison du ligand sur le deuxième domaine de la protéine de fusion, le troisième domaine ne peut pas exercer son activité d'activation d'une voie de signal se raccordant à une protéine Ras dans la cellule malgré la localisation membranaire, mais lors de la dissociation du ligand et du deuxième domaine, une modification de conformation s'effectue avec actions sur le troisième domaine, de sorte que le troisième domaine peut exercer son activité d'activation d'une voie de signal se raccordant à une protéine Ras dans la cellule et en ce que, en l'absence de la protéine de fusion au moins dans certaines conditions, une voie de signal se raccordant à une protéine Ras ne peut pas être activée dans la cellule.

6. Cellule selon la revendication 5,
**caractérisée en ce**
**que** la cellule est une cellule de levure.

7. Cellule selon la revendication 6,
**caractérisée en ce**
**que** la cellule est une cellule de levure sans paroi cellulaire.

8. Utilisation d'une cellule selon la revendication 5, dans un dosage *in vivo* pour détecter si un composé est en mesure de modifier une activité de liaison d'un segment de récepteur d'un récepteur nucléaire par rapport à un ligand, **caractérisée par** les étapes suivantes :
(a) mettre en contact le ligand en présence du composé avec des cellules selon la revendication 5, dans des conditions dans lesquelles le composé peut diffuser dans les cellules ou il est produit par les cellules et dans lesquelles en l'absence de la protéine de fusion, une voie de signal se raccordant à une protéine Ras ne peut pas être activée dans les cellules,
(b) examiner si et le cas échéant, dans quelle mesure, une activation de la voie de signal se raccordant à une protéine Ras est réalisée,
(c) comparer les résultats d'examen de l'étape (b) avec le résultat d'examen, qui est obtenu lors de la réalisation du dosage en l'absence du composé.

9. Utilisation selon la revendication 8, où dans l'étape (a), on met en oeuvre des cellules, dans lesquelles la voie de signal inactive, se raccordant à une protéine Ras, est une voie de signal, qui agit sur le cycle cellulaire et dont l'activation est essentielle pour la multiplication cellulaire, et l'étape (b) comprend le fait d'examiner si et le cas échéant dans quelle mesure, les cellules sont capables de multiplication dans les conditions citées, où dans le cas où la comparaison de l'étape (c) révèle qu'en présence du composé, une multiplication cellulaire plus importante se produit, une action agoniste du composé est montrée,
et dans le cas où la comparaison de l'étape (c) révèle qu'en présence du composé, une multiplication cellulaire plus faible se produit, une action antagoniste du composé est montrée.

10. Utilisation d'une cellule selon la revendication 5, dans un dosage *in vivo* pour détecter si un polypeptide ou une protéine présente une fonction de liaison d'un ligand d'un récepteur nucléaire, **caractérisée par** les étapes suivantes :
(a) mettre en contact des cellules selon la revendication 5 avec le ligand, dans des conditions dans lesquelles en l'absence de la protéine de fusion, une voie de signal se raccordant à une protéine Ras ne peut pas être activée dans les cellules, où la protéine de fusion contenue dans les cellules comprend un deuxième domaine, qui se compose du polypeptide ou de la protéine à examiner, et un troisième domaine, qui lors de la liaison du ligand au deuxième domaine, est en mesure d'activer la voie de signal inactive, se raccordant à une protéine Ras dans les cellules,
(b) examiner si une activation de la voie de signal se raccordant à une protéine Ras est réalisée,
où la détection de l'activation de la voie de signal se raccordant à une protéine Ras indique que le deuxième domaine de la protéine de fusion et de manière correspondante, le polypeptide ou la protéine à examiner, présente une fonction de liaison de ligand d'un récepteur nucléaire.

11. Kit à utiliser dans un dosage ou un procédé de criblage selon l'une des revendications 8 à 10,
**caractérisé en ce**
**que**
(a) il comprend des cellules selon la revendication 5, ainsi que
(b) le cas échéant, un ou plusieurs vecteurs de transformation ou de transfection, qui contiennent au moins une séquence d'ADN, qui code une protéine de fusion selon la revendication 1 ou 2,
(c) le cas échéant, des réactifs pour la transformation ou la transfection des cellules avec le vecteur de transformation ou de transfection,
(d) le cas échéant, des réactifs pour la détection de l'activation phénotypique de la voie de transduction de signal se raccordant à une protéine Ras dans ces cellules.

12. Kit à utiliser dans un dosage selon l'une des revendications 8 à 10,
**caractérisé en ce**
**qu'**il comprend les constituants suivants :
(a) des cellules selon la revendication 5,
(b) un vecteur de transformation ou de transfection, qui présente selon un agencement approprié
- une séquence d'ADN, qui code un premier domaine d'une protéine de fusion, telle que définie à la revendication 1 ou 2,
- une séquence d'ADN, qui code un troisième domaine d'une protéine de fusion, telle que définie à la revendication 1 ou 2, et
- un site d'insertion agencé de manière appropriée, pour l'insertion fonctionnelle d'une séquence d'ADN, qui code un deuxième domaine d'une protéine de fusion, telle que définie à la revendication 1 ou 2,
où après l'insertion d'une séquence d'ADN pour le deuxième domaine, le vecteur comprend un gène complet pour une protéine de fusion selon la revendication 1,
(c) le cas échéant, des réactifs pour la transformation ou la transfection des cellules avec le vecteur de transformation ou de transfection,
(d) le cas échéant, des réactifs pour la détection de l'activation phénotypique de la voie de transduction de signal se raccordant à une protéine Ras dans ces cellules.

13. Kit à utiliser dans un dosage selon la revendication 10,
**caractérisé en ce**
**qu'**il comprend les constituants suivants :
(a) des cellules selon la revendication 5,
(b) un vecteur de transformation ou de transfection, qui présente selon un agencement approprié
- une séquence d'ADN, qui code un premier domaine d'une protéine de fusion, telle que définie à la revendication 1 ou 2,
- une séquence d'ADN, qui code un troisième domaine d'une protéine de fusion, telle que définie à la revendication 1 ou 2, et
- un site d'insertion agencé de manière appropriée, pour l'insertion fonctionnelle d'une séquence d'ADN, qui code un deuxième domaine d'une protéine de fusion, telle que définie à la revendication 1 ou 2,
où après l'insertion d'une séquence d'ADN pour le deuxième domaine, le vecteur comprend un gène complet pour une protéine de fusion selon la revendication 1 ou 2, pour lequel une fonction de liaison de ligand d'un récepteur nucléaire est supposée,
(c) le cas échéant, des réactifs pour la transformation ou la transfection des cellules avec le vecteur de transformation ou de transfection,
(d) le cas échéant, des réactifs pour la détection de l'activation phénotypique de la voie de transduction de signal se raccordant à une protéine Ras dans ces cellules.

14. Kit selon la revendication 13,
comprenant en outre :
un vecteur de transformation ou de transfection, avec une construction comprenant :
- un site de liaison pour un facteur de transcription, dont l'activation est réalisée suite à une activation d'une voie de signal Ras spéciale, dont l'activation doit être détectée par le dosage,
- un promoteur minimal, et
- un site d'insertion approprié pour une expression dirigée par le promoteur minimal, pour l'insertion d'un gène pour une protéine reporter,
- où le promoteur minimal est activé suite à la liaison du facteur de transcription activé en son site de liaison.
